(19) Europäisches Patentamt / European Patent Office / Office européen des brevets

(11) **EP 3 315 119 B1**

(12) **EUROPEAN PATENT SPECIFICATION**

(45) Date of publication and mention
of the grant of the patent:
**04.09.2019 Bulletin 2019/36**

(51) Int Cl.:
*A61Q 11/00* *(2006.01)*    *A61K 8/06* *(2006.01)*
*A61K 8/22* *(2006.01)*    *A61K 8/31* *(2006.01)*

(21) Application number: **16195674.3**

(22) Date of filing: **26.10.2016**

(54) **KIT FOR TOOTH WHITENING COMPRISING AN ORAL COMPOSITION, A DELIVERY CARRIER AND AN ELECTROMAGNETIC RADIATION SOURCE**

KIT FÜR ZAHNAUFHELLUNG MIT EINER ORALEN ZUSAMMENSETZUNG, EINEM VERABREICHUNGSTRÄGER UND EINER ELEKTROMAGNETISCHEN STRAHLUNGSQUELLE

KIT DE BLANCHIMENT DES DENTS COMPOSÉ D'UNE COMPOSITION ORALE, D'UN EXCIPIENT ET D'UNE SOURCE DE RAYONNEMENT ÉLECTROMAGNÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**02.05.2018 Bulletin 2018/18**

(73) Proprietor: **The Procter & Gamble Company Cincinnati, OH 45202 (US)**

(72) Inventors:
• **RAJAIAH, Jayanth**
  **Cincinnati, OH Ohio 45202 (US)**
• **SAGEL, Paul Albert**
  **Cincinnati, OH Ohio 45202 (US)**

(74) Representative: **P&G Patent Germany**
**Procter & Gamble Service GmbH**
**Sulzbacher Straße 40**
**65824 Schwalbach am Taunus (DE)**

(56) References cited:
**WO-A1-2010/098761    WO-A1-2013/093743**
**US-A1- 2007 280 894**

EP 3 315 119 B1

**Description**

FIELD OF THE INVENTION

The present invention relates to a kit for whitening teeth. Said kit comprises a delivery carrier, a multi-phase oral composition for whitening teeth in form of a water-in-oil emulsion and an electromagnetic radiation source capable of directing electromagnetic radiation with one or more wavelengths in the range from 200nm to 1700 nm toward at least one tooth. The present oral compositions comprise a hydrophobic phase, an aqueous phase and a bleaching agent as an oral care active agent in the aqueous phase, wherein the hydrophobic (e.g. oil) phase is in predominant proportion relative to the aqueous phase, in particular the oral composition comprises a very low level of aqueous phase, e.g. up to about 5% by weight of the oral composition. Further, the oral composition comprises a very low level of oral care active agent, namely up to 0.1% by weight of the oral composition. In addition, the invention relates to the use of said kit and a method of whitening teeth.

BACKGROUND OF THE INVENTION

Dental products by which various cosmetic and/or therapeutic actives are delivered to teeth and the oral cavity are known. Examples of such products include: brushing aids such as dentifrice products for delivery of oral care actives such as polyphosphates or fluorides; mouthwashes containing breath fresheners or antibacterial actives; and whitening strips for the delivery of bleaching actives to the teeth. In particular the use of a dental strip has been recognized as a convenient and inexpensive way to deliver cosmetic and therapeutic benefits to the teeth and mucosal surfaces of the oral cavity. For example, dental whitening strips, where a whitening composition is applied to a strip and thereafter applied to the teeth to achieve sustained contact between the teeth and the whitening composition, are known. U.S. Pat. Nos. 6,136,297; 6,096,328; 5,894,017; 5,891,453; and 5,879,691, all to Sagel, et al., and U.S. Pat. Nos. 5,989,569 and 6,045,811 both to Dirksing, et al., all assigned to The Procter & Gamble Company. Some of those composition use emulsions for the composition to be applied, see e.g. WO 2005/058267 A1, WO 2005/058268 A1, U.S. Pub. No 2007/0280894 A1 to Romano et al., all assigned to The Procter & Gamble Company.

Despite the above known approaches for the treatment of oral conditions, especially for the whitening of teeth, a need still exists for providing products with both improved bleaching efficacy, increased speed of whitening, decreased tooth-sensitivity, and/or decreased oral soft tissue irritation. The prior art has generally attempted to address improved bleaching efficacy and/or increased speed of whitening by increasing the level of the bleaching agent in the compositions. This approach, however, presents several problems. First the participant may experience increased irritation and/or sensitivity which may be associated with using an increased amount of a bleaching agent. Furthermore, some regulatory authorities and legislation in various geographies throughout the world do not allow bleaching agents to be used in products at levels above certain concentrations. Therefore, despite the above known approaches for the treatment of oral conditions, especially for the whitening of teeth, a need still exists for providing products with improved bleaching efficacy, increased speed of whitening, decreased tooth-sensitivity, and/or decreased oral soft tissue irritation. The present invention overcomes some of the limitations of the prior art, and relates to a multi-phase, preferably water-in-oil composition comprising a bleaching agent, an aqueous phase and a hydrophobic phase, wherein the hydrophobic phase is in predominant proportion relative to the aqueous phase.

SUMMARY OF THE INVENTION

Without being bound to a theory it was surprisingly found that bleaching agents are effective in very low concentration, if presented in an oral composition as disclosed herein.

According to one aspect there is provided a kit for whitening teeth comprising

I) a multi-phase oral composition in the form of a water-in-oil emulsion for whitening teeth, comprising:

a) from 0.01 to 1% by weight of an aqueous phase;
b) at least 99% by weight of a hydrophobic phase; and
c) at least one oral care active agent comprised in the aqueous phase;
wherein the active agent is a bleaching agent and the final concentration of the bleaching agent is up to or less than 0.1% by weight of the oral composition;

II) a delivery carrier, suitable to carry and the oral composition; and
III) an electromagnetic radiation source capable of directing electromagnetic radiation with one or more wavelength in the range from 200 to 1700nm toward at least one tooth.

The present specification also discloses the use of said kit for whitening teeth and/or removing stain from tooth surfaces. In addition the invention further relates to a method of whitening teeth, as defined in claims 21 to 23.

The present invention is used to deliver whitening benefits to the oral cavity by directly applying the oral composition to the teeth. The oral composition may be applied via a delivery carrier, such as a strip or film of material, dental tray, sponge material or mixtures thereof. The delivery carrier is attached to the teeth via the oral compositions herein or the adhesion function can be provided independent of the present oral compositions herein (e.g. can be provided via a separate adhesive composition used with the present oral compositions and delivery carrier).

BRIEF DESCRIPTION OF THE DRAWINGS

Fig. 1 is a perspective view of a delivery system 10 comprising a strip of material 12 having rounded corners upon which in a second layer 14 the present oral compositions are coated;

Fig. 2 is a cross-sectional view, taken along section line 3-3 of Fig. 1, showing an example of the strip 12;

Fig. 3 is a cross-sectional plan view, showing the delivery system 10 attached to the teeth 22 by means of the second layer 14 oral composition located between the teeth 22 and the strip of material 12;

Fig. 4 is a cross-sectional elevation view of a tooth, taken along section line 6-6 of Fig. 3, showing the delivery system 10 adhesively attached to the teeth 22;

FIG. 5 shows a dental tray 30 suitable to be used with the oral composition of the present invention;

Fig. 6 shows a device for delivering electromagnetic radiation with a peak intensity wavelength of about 455nm to a transparent mouthpiece to help position the electromagnetic radiation reproducibly toward the tooth surface;

Fig. 7 shows the bleaching efficacy on a natural tooth surface after 14 treatments using an oral composition of the present invention (Example-IA delivered on a strip and used with electromagnetic radiation having a peak intensity wavelength of about 455nm).

DETAILED DESCRIPTION OF THE INVENTION

While the specification concludes with claims particularly pointing out and distinctly claiming the invention, it is believed that the present invention will be understood better from the following description of embodiments, taken in conjunction with the accompanying drawings, in which like reference numerals identify identical elements.

The oral compositions herein are useful for topical application, in particular for topical application in the mouth. I.e. the composition of the present invention is a composition for use in oral care, thus, being an oral composition; by "oral composition" as used herein is meant a product which in the ordinary course of usage is not intentionally swallowed for purposes of systemic administration of therapeutic agents, but is retained in the oral cavity for a sufficient time to contact the dental surfaces or oral tissues for intended purposes, e.g. for whitening efficacy. Examples of oral compositions include dentifrice, tooth gel, subgingival gel, mouth rinse, mousse, foam, mouth spray, lozenge, chewable tablet, chewing gum, tooth whitening strip coatings, floss coatings, breath freshening dissolvable strip coatings, or denture care or adhesive products. The oral care composition may also be incorporated onto strips or films for direct application or attachment to oral surfaces.

The term "dentifrice", as used herein, includes tooth or subgingival -paste, gel, or liquid formulations unless otherwise specified. The dentifrice composition may be a single phase composition or may be a combination of two or more separate dentifrice compositions. The dentifrice composition may be in any desired form, such as deep striped, surface striped, multilayered, having a gel surrounding a paste, or any combination thereof. Each dentifrice composition in a dentifrice comprising two or more separate dentifrice compositions may be contained in a physically separated compartment of a dispenser and dispensed side-by-side.

The oral compositions as disclosed herein are multi-phase compositions for whitening teeth, preferably comprising a water-in-oil emulsion. The oral compositions as disclosed herein are preferably water-in-oil emulsions, i.e. the compositions are in the form of an emulsion comprising an aqueous phase and a hydrophobic phase, wherein the hydrophobic phase is the continuous phase, and the aqueous phase comprises at least one oral care active, such as a whitening agent.

The term "immiscible" as used herein means less than 1 part by weight of the substance dissolves in 99 parts by weight of a second substance.

The term "phase" as used herein means a physically distinct region or regions, which may be continuous or discontinuous, having one or more properties that are different from another phase. Non-limiting examples of properties that may be different between phases include composition, viscosity, solubility, hydrophobicity, hydrophilicity, and miscibility.

The term "multi-phase composition" as used herein comprises a mixture of two or more phases that are immiscible with each other. The phases may be continuous, discontinuous, or combinations thereof. Examples of multi-phase compositions include emulsions, in particular water in oil emulsions. Examples of multi-phase compositions also include oil-in-water emulsions, water-in-oil-in-water emulsions, and oil-in-water-in-oil emulsions. Examples of multi-phase compositions also include compositions where the phases are multi-continuous including bi-continuous, layered, striped, marbled, ribbons, swirled, and combinations thereof.

The term "emulsion" as understood herein is an example of a multi-phase composition wherein 1) at least one of the phases is discontinuous and 2) at least one of the phases is continuous . Examples of emulsions include droplets of water dispersed in oil. In this example the water and oil would be mutually immiscible with each other, water would be the discontinuous phase, and the oil would be the continuous phase.

The term "water-in-oil emulsion" as understood herein is an example of an emulsion wherein 1) the discontinuous phase is aqueous, and 2) the continuous phase is hydrophobic.

The term "aqueous phase" as understood herein is at least one phase that comprises water and a bleaching agent, and is immiscible with the hydrophobic phase. In certain embodiments, each region of the aqueous phase contains at least 2% of the bleaching agent by weight of the aqueous phase. Optionally the aqueous phase may further comprise ingredients that are water soluble, water miscible, or combinations thereof, such as for example water soluble solvents, alcohol, polyethylene glycol, carbopol, , etc. or mixtures thereof. In contrast, if in some embodiments immiscible fillers are added to the aqueous phase, the percentage of the aqueous phase in the composition is calculated by excluding the immiscible filler.

The term "hydrophobic phase" as understood herein means all components of the composition that are immiscible with the aqueous phase. Optionally the hydrophobic phase may further comprise ingredients that are soluble, miscible or combinations thereof in the hydrophobic phase, such as for example hydrocarbon solvents dissolved into the hydrophobic phase, polyethylene dissolved into the hydrophobic phase, microcrystalline wax dissolved into the hydrophobic phase, or mixtures thereof.

The term "delivery carrier" as used herein comprises a material or an appliance that is used to hold the oral composition against the tooth surface. Delivery carriers may be strips or dental trays.

The term "strip" as used herein comprises a material 1) whose longest dimension length is generally greater than its width, and 2) whose width is generally greater than its thickness. Strips may be rectangular, arched, curved, semi-circular, have rounded corners, have slits cut into it, have notches cut into it, bent into three dimensional shapes, or combinations thereof. Strips may be solid, semi-solid, textured, moldable, flexible, deformable, permanently deformable, or combinations thereof. Strips may be made from plastic sheets including polyethylene, or wax sheets. Examples of strips include a piece of polyethylene about 66mm long, 15mm wide, and 0.0178mm thick. Examples of permanently deformable strips include a piece of casting wax sheet about 66mm long, 15mm wide, and 0.4mm thick.

By "safe and effective amount" as used herein is meant an amount of a component, high enough to significantly (positively) modify the condition to be treated or to effect the desired whitening result, but low enough to avoid serious side effects (at a reasonable benefit/risk ratio), within the scope of sound medical/dental judgment. The safe and effective amount of a component, will vary with the particular condition being treated, the age and physical condition of the patient being treated, the severity of the condition, the duration of treatment, the nature of concurrent therapy, the specific form employed, and the particular vehicle from which the component is applied.

By "a sufficient period of time to achieve whitening" as used herein is meant that the oral composition is used or worn by the participant or the participant is instructed to use or wear the oral composition for greater than about 10 seconds, greater than about 1 minute, greater than about 5 minutes, greater than about 10 minutes, in particular from about 1, 5, 10, or 15 minutes to about 20, 30, 60, 120 minutes per application or any other numerical range which is narrower and which falls within such broader numerical range, as if such narrower numerical ranges were all expressly written herein, preferably from about 2.5 minutes to about 12 hours (e.g. overnight treatment), or from about 3 minutes to about 180 minutes, more preferred from about 5 minutes to about 60 minutes, more preferred from about 10 minutes to about 60 minutes. The treatments may be applied from about 1, 2, or 3 times a day to about 4, 5, 6 or 7 times a day. The treatments may be applied for from about 1, 2, 3, 4, 5, 6, or about 7 days to about 8, 9, 10, 11, 12, 13, 14, 21, or 28 days or any other numerical range which is narrower and which falls within such broader numerical range, as if such narrower numerical ranges were all expressly written herein. Further, the length of treatment to achieve the desired benefit, for example, tooth whitening, may last for a specified period of time, which may be repeated if necessary, for example from about one day to about six months, in particular from about one day to about 28 days, or from about 7 to about 28 days. The optimal duration and frequency of application will depend on the desired effect, the severity of any condition being treated, the health and age of the user and like considerations.

The term "dispenser", as used herein, means any pump, tube, or container suitable for dispensing oral compositions.

By "cm" as used herein is meant centimeter. By "mm" as used herein is meant millimeter. By "$\mu$m" or "microns" as used herein is meant micrometer.

The term "wt %" as used herein means percentage by weight.

All percentages and ratios used herein after are by weight of total oral composition unless otherwise indicated. All percentages, ratios, and levels of ingredients referred to herein are based on the actual amount of the ingredient, and do not comprise solvents, fillers, or other materials with which the ingredient may be combined as a commercially available product, unless otherwise indicated.

For example, an oral composition that contains 0.2857% of an aqueous solution of 35% hydrogen peroxide (H2O2) and 99.7143% petrolatum would mean this oral composition contains 0.2857% of an aqueous phase (namely the aqueous solution of 35% H2O2), 99.7143% of a hydrophobic phase (namely the petrolatum), and 0.099995% of an oral care active (namely the H2O2 in the aqueous phase). As another example, an oral composition that contains 0.2857% of an aqueous solution of 35% hydrogen peroxide (H2O2), 89.7143% petrolatum, and 10% silica dispersed in the petrolatum would mean this oral composition contains 0.2857% of an aqueous phase (namely the aqueous solution of 35% H2O2), 99.7143% of a hydrophobic phase (namely the petrolatum and silica which are both immiscible with the aqueous phase), 0.099995% of an oral care active (namely the H2O2 in the aqueous phase), and 10% of a filler (namely the silica) which is part of the hydrophobic phase. This would also mean that this composition has a ratio of the concentration in weight percent of bleaching agent present in the aqueous phase to the net concentration in weight percent of bleaching agent present in the overall oral composition of 350.02 (namely 35% divided by 0.099995%)

As yet another example, an oral composition that contains 0.2857% of an aqueous solution of 35% hydrogen peroxide (H2O2), 99.6143% petrolatum, and 0.1% cross-linked siloxane particles dispersed in the aqueous phase would mean this oral composition contains 0.2857% of an aqueous phase (namely the aqueous solution of 35% H2O2, 99.7143% of a hydrophobic phase (namely the petrolatum and cross-linked siloxane particles which are both immiscible with the aqueous phase), 0.099995% of an oral care active (namely the H2O2 in the aqueous phase), and 0.1% of a filler (namely the cross-linked siloxane particles) which is part of the hydrophobic phase. This would mean that this composition has a ratio of the concentration in weight percent of bleaching agent present in the aqueous phase to the net concentration in weight percent of bleaching agent present in the overall oral composition of 350.02 (namely 35% divided by 0.099995%).

All measurements referred to herein are made at about 22°C (i.e. room temperature) unless otherwise specified.

As used herein, the word "about" means +/- 10 percent.

As used herein, the word "or" when used as a connector of two or more elements is meant to include the elements individually and in combination; for example X or Y, means X or Y or both.

As used herein, the word "comprise," and its variants, are intended to be non-limiting, such that recitation of items in a list is not to the exclusion of other like items that may also be useful in the materials, oral compositions, devices, and methods of this invention. This term encompasses the terms "consisting of' and "consisting essentially of'.

As used herein, the word "include," and its variants, are intended to be non-limiting, such that recitation of items in a list is not to the exclusion of other like items that may also be useful in the materials, oral compositions, devices, and methods of this invention.

As used herein, the words "preferred", "preferably" and variants refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

"Active and other ingredients" useful herein may be categorized or described herein by their cosmetic and/or therapeutic benefit or their postulated mode of action or function. However, it is to be understood that the active and other ingredients useful herein can, in some instances, provide more than one cosmetic and/or therapeutic benefit or function or operate via more than one mode of action. Therefore, classifications herein are made for the sake of convenience and are not intended to limit an ingredient to the particularly stated function(s) or activities listed.

The term "teeth", as used herein, refers to natural teeth as well as artificial teeth or dental prosthesis and is construed to comprise one tooth or multiple teeth. The term "tooth surface" as used herein, refers to natural tooth surface(s) as well as artificial tooth surface(s) or dental prosthesis surface(s) accordingly.

The term "orally acceptable carrier" comprises one or more compatible solid or liquid excipients or diluents which are suitable for topical oral administration. By "compatible," as used herein, is meant that the components of the oral composition are capable of being commingled without interaction in a manner which would substantially reduce the oral composition's stability and/or efficacy.

Citation of any reference is not an admission regarding any determination as to its availability as prior art to the claimed invention. The essential and optional components of the present oral compositions are described in the following paragraphs:

The embodiments of the subject invention are oral compositions in form of a water-in-oil type emulsion.

Oral Compositions in form of an emulsion

The oral compositions as disclosed herein are water-in-oil emulsions.

The discontinuous phase is preferably dispersed in the continuous phase as small droplets. The oral compositions may be micro-emulsions and/or macro-emulsions. The number-average droplet diameter may be in the range of from about 0.001 $\mu$m to about 10,000 $\mu$m, or from about 0.01 $\mu$m to about 1000 $\mu$m, in particular in the range from about 0.1 $\mu$m to about 1000 $\mu$m, more particular in the range from about 1 $\mu$m to about 100 $\mu$m. The components of the oral composition are chosen to allow for the release of the bleaching agent located in the aqueous phase readily from the oral composition. The maximum amount of aqueous phase is 5% by weight of the oral composition.

Without being bound by theory it is believed that when the present invention, in the form of a water-in-oil emulsion, is brought into contact with a tooth surface, the aqueous phase and the components of the aqueous phase may migrate to the tooth surface. That means, the bleaching agent may be covered, protected against environmental influence and thereby stabilized by the hydrophobic phase of the oral composition until use and potentially by the hydrophobic phase in form of a film or layer during use. Thereby, the active effect may be applied to the tooth surface and the active agent, e.g. the bleaching agent may be potentially shielded against the oral environment during use. Thereby the efficacy of a whitening oral composition may be enhanced and/or accelerated.

Without further being bound by theory, the present invention may improve the delivery of the whitening agent to the tooth surface and thus the whitening performance due to the partial hydrophobic and partial hydrophilic nature of the oral composition. Due to the driving force resulting therefrom the bleaching agent present in the aqueous phase may be driven towards the tooth surface. Thereby increased speed of whitening and increased efficacy of the bleaching agent may be achieved, even though surprisingly low total levels of the bleaching agent are used. The present invention, therefore, at a given net concentration, such as 0.1% by weight or below of a bleaching agent, delivers a surprisingly high level of whitening efficacy, may require fewer applications to get the same degree of whitening, or may require a lower gel load to get the same degree of whitening.

In addition, retention of the oral composition on the tooth surfaces may be improved as the hydrophobic phase resists salivary dilution and salivary enzymes which can decompose the peroxide. Even furthermore, the hydrophobic phase does not dehydrate the teeth creating an outward flux of water created by many hydrophilic compositions containing hydrophilic adhesives such as polycarboxylic acid. Since the hydrophobic phase does not dehydrate the teeth it may result in a surprisingly low level of tooth sensitivity even while delivering a surprisingly high level of whitening efficacy.

In addition, the predominant proportion of the hydrophobic phase may provide further advantages. For example the hydrophobic phase represents a stable matrix for ingredients and compounds which are soluble in the hydrophobic phase. For example, many flavor ingredients usually used in oral compositions are soluble in the hydrophobic phase. That means the flavor ingredients may be protected from any influence of the active agent, e.g. the bleaching agent, in the oral composition. In addition, during use of the oral composition at the tooth surface at least part of the hydrophobic phase may be located - without being bound by theory- towards the soft oral tissues, such as the mucosa, thereby presenting the ingredients which are present in the hydrophobic phase, such as flavor compounds, to the oral cavity. In addition, the hydrophobic phase may shield the active agent, such as the bleaching agent against any influence from the oral cavity, such as dilution by saliva. The shielding effect may also apply to the tooth surface(s) themselves, wherein the hydrophobic phase may provide greater hydration of the teeth surfaces.

The oral compositions as disclosed herein may be in the form of a liquid, viscous liquid, gel, semi-solid, solid, viscoelastic liquid, viscoelastic gel, sol, viscoelastic solid, solid, or any combination thereof.

Aqueous Phase

The present oral compositions comprise a safe and effective amount of an aqueous phase. In addition, the aqueous phase of the present oral composition includes one or more actives or ingredients. The amount of the aqueous phase may be from about 0.002%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, or 1% by weight of the oral composition to about 5%, 4%, 3%, 2%, 1.5%, 1.4%, 1.3%, 1.2%, 1.0%, 0.9%, 0.8%, 0.7%, 0.6%, 0.5%, 0.4%, 0.3%, 0.2% or 0.1, or 0.01% by weight of the oral composition or any other numerical range which is narrower and which falls within such broader numerical range, as if such narrower numerical ranges were all expressly written herein. In particular, the oral composition comprises from about 0.002% to about 5%, preferably from about 0.01% to about 2% by weight, more preferred from about 0.01% to about 1% by weight, more preferred from about 0.01% to about 0.95% by weight of the oral composition of the aqueous phase or any other numerical range which is narrower and which falls within such broader numerical range, as if such narrower numerical ranges were all expressly written herein. Alternatively, the amount of the aqueous phase may be less than about 5%, 4%, 3%, 2%, 1.5%, 1.4%, 1.3%, 1.2%, 1.0%, 0.9%, 0.8%, 0.7%, 0.6%, 0.5%, 0.4%, 0.3%, 0.2% or 0.1% by weight of the oral composition. In particular, the amount of the aqueous phase maybe less than about 1.0% or 0.9% by weight of the oral composition.

The aqueous phase may preferably comprise water, polyalkylene glycols with molecular weights from about 200 to about 20,000, humectants, and mixtures thereof. Humectants generally include edible polyhydric alcohols such as glycerin, sorbitol, xylitol, butylene glycol, polyethylene glycol, and propylene glycol, and mixtures thereof. The aqueous phase may comprise at least about 10% by weight of the aqueous phase water, preferably at least about 20% by weight of the aqueous phase water, and more preferred the aqueous phase is water together with the bleaching agent.

Bleaching Agent

The level of bleaching agent is based on the available oxygen or chlorine respectively that the molecule is capable of providing to bleach the stain. The present oral compositions comprise a safe and effective amount of a bleaching agent, wherein the net amount of bleaching agent is 0.1% by weight of the oral composition at maximum. Preferably the bleaching agent level is less than 0.1% by weight of the oral composition, in some instances less than 0.09%, 0.08%, 0.07%, 0.06%, 0.05%, 0.04%, 0.03%, 0.02%, 0.01%, or 0.001% by weight of the oral composition, preferably from about 0.01% to about 0.099995%, more preferred from about 0.01% to about 0.095%, more preferred from about 0.05% to about 0.09% by weight of the oral composition or any other numerical range which is narrower and which falls within such broader numerical range, as if such narrower numerical ranges were all expressly written herein. Surprisingly, the bleaching agent is significantly effective when used even at the low levels in the multi-phase compositions as disclosed herein, preferably in the form of water-in-oil emulsions.

The bleaching agent of the oral compositions of the present invention is present in the aqueous phase from about 2%, 5%, 8.75%, 10%, 15%, 17.5%, 20%, 25%, 30%, 35% or 45% to about 50%, 45%, 40%, 35%, 30%, 25%, 20%, 17.5%, 15%, 10%, 8.75% or 5% by weight of the aqueous phase, or any other numerical range which is narrower and which falls within such broader numerical range, as if such narrower numerical ranges were all expressly written herein Preferably the oral composition comprises bleaching agent, in the aqueous phase from about 8.75%, 10%, 15%, 17.5%, 20%, 25% or 30% to about 35%, 30%, 25%, 20%, 17.5%, 15% or 10% by weight of the aqueous phase, more preferred the oral composition comprises bleaching agent in the aqueous phase from about 17.5%, 20%, 25% or 30% to about 35%, 30%, 25% or 20% by weight of the aqueous phase or any other numerical range which is narrower and which falls within such broader numerical range, as if such narrower numerical ranges were all expressly written herein.

Ratio of concentrations of bleaching agent

The oral compositions of the present invention deliver a high ratio of the concentration in weight percent of bleaching agent present in the aqueous phase to the net concentration in weight percent of bleaching agent present in the overall oral composition because they have a high concentration in weight percent of bleaching agent present in the aqueous phase combined with a relatively low concentration in weight percent of bleaching agent present in the overall oral composition. In other words, the amount of aqueous phase is very small in the overall composition and so even a high concentration of bleaching agent in the aqueous phase results in a low concentration of bleaching agent in the net composition. Without being bound by theory, this surprising combination of seemingly contradictory parameters in the present invention delivers the bleaching agent to the tooth surface with a high driving force even when the overall concentration or net amount of bleaching agent delivered to the tooth surface is low. Consequently, 1) The high driving force delivers a surprisingly high level of bleaching efficacy and/or bleaching speed, while 2) The low overall concentration or low amount of bleaching agent delivered to the tooth surface may help reduce tooth sensitivity.

The ratio of the concentration in weight percent of bleaching agent present in the aqueous phase to the net concentration in weight percent of bleaching agent present in the overall oral composition may be from about 50000, 35000, 20000, 17500, 10000, 5000, 3500, 2000, 1750, 1160, 1000, 875, 700, 580, 500, 430, 400, 380, 350, 200, 175, 100, 50 or 25 to about 20, 25, 50, 100, 175, 200, 350, 380, 430, 400, 500, 580, 700, 875, 1000, 1160, 1750, 3500, 5000, 10000, 17500, 20000, 35000 or 50000, preferably, the ratio of the concentration in weight percent of bleaching agent present in the aqueous phase to the net concentration in weight percent of bleaching agent present in the overall oral composition is from about from about 10000, 5000, 3500, 2000, 1750, 1160, 1000, 875, 700, 580, 500, 430, 400, 380, 350, 200, 175, 100, 50 or 25 to about 20, 25, 50, 100, 175, 200, 350, 380, 430, 400, 500, 580, 700, 875, 1000, 1160, 1750, 3500 or 5000, more preferred, the ratio of the concentration in weight percent of bleaching agent present in the aqueous phase to the net concentration in weight percent of bleaching agent present in the overall oral composition is from about 1750, 1160, 1000, 875, 700, 580, 500, 430, 400, 380, 350, 200, 175, 100, 50 or 25 to about 20, 25, 50, 100, 175, 200, 350, 380, 430, 400, 500, 580, 700, 875, 1000 or 1160 or any other numerical range which is narrower and which falls within such broader numerical range, as if such narrower numerical ranges were all expressly written herein.

Even more preferred the ratio of the concentration in weight percent of bleaching agent present in the aqueous phase to the net concentration in weight percent of bleaching agent present in the overall oral composition may be at least or more than about 20, 25, 50, 100, 175, 200, 350, 380, 430, 400, 500, 580, 700, 875, 1000, 1160, 1750, 3500, 5000, 10000, 17500, 20000, or 35000, preferably the ratio may be at least or more than about 20, 25, 50, 100, 175, 200, 350, 380, 430, 400, 500, 580, 700, 875, 1000, 1160, or 1750, more preferred the ratio may be at least or more than about 50, 100, 175, 200, 350, 380, 430, 400, 500, 580, 700, 875, 1000, 1160, or 1750 and even more preferred the ratio of the net concentration in weight percent of bleaching agent present in the aqueous phase to the net concentration in weight percent of bleaching agent present in the overall oral composition is at least or more than about 50, 100, 175, or 350.

Suitable bleaching agents include agents that provide bleaching effects, stain bleaching effects, stain removal effects, stain color change effects or any other effect, which changes, in particular brightens the tooth color. For example bleaching agents comprise a source of peroxide radicals. In addition, bleaching agents may include peroxides, metal chlorites, metal hypochlorites, perborates, percarbonates, peroxyacids, persulfates, compounds that form the preceding compounds in situ, and combinations thereof. Suitable example peroxide compounds include hydrogen peroxide ($H_2O_2$), urea peroxide, calcium peroxide, carbamide peroxide, and mixtures thereof. A preferred bleaching agent is hydrogen peroxide. Suitable example metal chlorites include calcium chlorite, barium chlorite, magnesium chlorite, lithium chlorite, sodium chlorite, potassium chlorite, and mixtures thereof. Additional bleach agents also include hypochlorite and chlorine dioxide. The starting bleaching agent material can be a solid material or already be solved in an aqueous solution. If solid material is used the bleaching agent is dissolved in the aqueous phase of the oral composition as disclosed herein. If aqueous material is used the active agent is mixed with the aqueous phase of the oral composition.

The bleaching agents of the present oral composition may be stabilized against degradation due to the shielding effect of the hydrophobic phase. In the oral composition of the present invention at least about 10% of the bleaching agent, e.g. the hydrogen peroxide comprised in the oral composition may be present after 180 days storage at 30°C, when the oral composition is covered to light exposure. In particular at least about 25% of the hydrogen peroxide, more particular at least about 50% of the hydrogen peroxide, even more particular at least about 75% of the hydrogen peroxide may be present after 180 days storage of the oral composition at 30°C, when the oral composition is protected from light exposure.

Optional Stabilizing Agent for the Bleaching Agent

The oral compositions of the current invention may comprise a stabilizing agent. The bleaching agent may be further stabilized against degradation by the oral composition. Therefore stabilizing agents may be added to the aqueous phase of the present oral composition. In particular, if hydrogen peroxide is used stabilizing agents may be added. Suitable stabilizing agents are for example ortho-phosphoric acid, phosphate(s), such as sodium hydrogen phosphate, pyrophosphate(s), organophosphonate(s), Ethylenediaminetetraacetic acid, Ethylenediamine-N,N'-diacetic acid, Ethylenediamine-N,N'-disuccinic acid, potassium stannate, sodium stannate, tin salts, zinc salts, salicylic acid, 1-Hydroxyethylidene-1,1-diphosphonic acid, and a combination thereof. In particular, stabilizers may be used which show additional oral care effects, such as anti-tartar effect, produced by pyrophosphate(s) or organophosphonate(s). Optionally, the bleaching agent stabilizing agent is present in the oral composition of the present invention in an amount from about 0.0000001%, 0.000001%, or 0.00001%, to about 0.00001%, 0.0001%, or 0.01% by weight of the oral composition or any other numerical range which is narrower and which falls within such broader numerical range, as if such narrower numerical ranges were all expressly written herein. Optionally, the bleaching agent stabilizing agent may be present in the oral composition of the present application in an amount from about 0.0001%, or 0.01% to about 0.01%, 0.1% or about 1% by weight of the aqueous phase or any other numerical range which is narrower and which falls within such broader numerical range, as if such narrower numerical ranges were all expressly written herein.

A stabilizing agent may also be a chelant or may include chelants. The chelant may be a copper, iron and/or manganese chelant, or a mixture thereof. Suitable chelants may be selected from: diethylene triamine pentaacetate, diethylene triamine penta(methyl phosphonic acid), ethylene diamine-N'N'-disuccinic acid, ethylene diamine tetraacetate, ethylene diamine tetra(methylene phosphonic acid), hydroxyethane di(methylene phosphonic acid), and any combination thereof. A suitable chelant may be selected from ethylene diamine-N'N'-disuccinic acid (EDDS), hydroxyethane diphosphonic acid (HEDP) or mixtures thereof. The stabilizer may comprise ethylene diamine-N'N'-disuccinic acid or salt thereof. The ethylene diamine-N'N'-disuccinic acid may be in S,S enantiomeric form. The stabilizer may comprise 4,5-dihydroxy-m-benzenedisulfonic acid disodium salt, glutamic acid-N,N-diacetic acid (GLDA) and/or salts thereof, 2-hydroxypyridine-1-oxide, Trilon PTM available from BASF, Ludwigshafen, Germany. Suitable chelants may also be calcium carbonate crystal growth inhibitors. Suitable calcium carbonate crystal growth inhibitors may be selected from the group consisting of: 1-hydroxyethanediphosphonic acid (HEDP) and salts thereof; N,N-dicarboxymethyl-2-aminopentane-1,5-dioic acid and salts thereof; 2-phosphonobutane-1,2,4-tricarboxylic acid and salts thereof; and any combination thereof.

A stabilizer may comprise a calcium carbonate crystal growth inhibitor, such as 1-hydroxyethanediphosphonic acid (HEDP) and salts thereof; N,N-dicarboxymethyl-2-aminopentane-1,5-dioic acid and salts thereof; 2-phosphonobutane-1,2,4-tricarboxylic acid and salts thereof; and any combination thereof.

A stabilizer may comprise a hydroxamate chelant. By 'hydroxamate' we herein mean hydroxamic acid or the corresponding salt. A preferred hydroxamate chelant is coco hydroxamic acid (Axis House RK 853).

Hydrophobic Phase

The present oral compositions comprise a safe and effective amount of a hydrophobic phase. The hydrophobic phase is in predominant proportion relative to the aqueous phase present in the oral composition. As used herein "predominant proportion" means that the percent by weight of the oral composition of the hydrophobic phase is in excess relative to the percent by weight of the oral composition of the aqueous phase.

The present oral compositions comprise a safe and effective amount of a hydrophobic phase, wherein the hydrophobic phase is at least about 95%, 96%, 97%, 98%, 99%, 99.1% or 99.5% by weight of the oral composition. In particular, the hydrophobic phase may be at least about 99%, 99.1% or 99.5% by weight of the oral composition. Preferably, the hydrophobic phase may be at least about 99.1% or 99.5% by weight of the oral composition, more preferred the hydrophobic phase may be at least about 99.5% by weight of the oral composition.

The hydrophobic phase may be inert or is at least partially inert and does preferably not interact or only minimally interact with the bleaching agent and other optional ingredients, if present in the oral composition.

A suitable hydrophobic phase for the oral compositions as disclosed herein may have an octanol/water partition coefficient (log $P_{ow}$) of greater than about 2, 3, 4, or 5, preferably greater than about 5.5, more preferred the hydrophobic phase shows a $P_{ow}$ > about 6 or any other numerical range which is narrower and which falls within such broader numerical range, as if such narrower numerical ranges were all expressly written herein.

The drop melting point of a suitable hydrophobic phase may be in the range of from about 40°C to about 70°C, in particular in the range of from about 50° to about 65°C, more particular in the range of from about 50°C to about 60°C or any other numerical range which is narrower and which falls within such broader numerical range, as if such narrower numerical ranges were all expressly written herein as measured according to ASTM method D127-08. A cone penetration consistency value of a hydrophobic phase used in the present oral compositions may be in the range of from about 100 to about 300, in particular in the range of from about 150 to about 250, more particular in the range of from about 170 to about 200 or any other numerical range which is narrower and which falls within such broader numerical range, as if such narrower numerical ranges were all expressly written herein as measured according to ASTM method D937-07. A suitable density of the hydrophobic phase used in the present oral compositions is in the range of from about 0.8 g/cm3 to about 1.0 g/cm3, preferably in the range of from about 0.85 g/cm3 to about 0.95 g/cm3, more preferred about 0.9 g/cm3 or any other numerical range which is narrower and which falls within such broader numerical range, as if such narrower numerical ranges were all expressly written herein.

A suitable hydrophobic phase is non-toxic oil, in particular non-toxic edible oil. In particular, the hydrophobic phase is selected from the group consisting of non-toxic edible oils, saturated or unsaturated fatty alcohols, aliphatic hydrocarbons, long chain triglycerides, fatty esters, and mixtures thereof. In addition, the hydrophobic phase may also comprise silicones, polysiloxanes, and mixtures thereof. Preferably the hydrophobic phase is selected from mineral oil, in particular petrolatum and mixtures thereof, more preferred petrolatum. Examples of petrolatum include Snow White Pet - C from Calumet Specialty Products (Indianapoli, IN), G-2191 from Sonneborn (Parsippany, NJ), G-2218 from Sonneborn, G-1958 from Sonneborn, and mixtures thereof.

The aliphatic hydrocarbons may contain from about 10, 12, 14, or 16 to about 16, 18, 20, 22, 24, 26, 28, 30, 36, 40 carbon atoms such as decane, 2 ethyldecane, tetradecane, isotetradecane, hexadecane, eicosane, and mixtures thereof. Long chain triglycerides include vegetable oils, fish oils, animal fats, hydrogenated vegetable oils, partially hydrogenated vegetable oils, semi-synthetic triglycerides, synthetic triglycerides, and mixtures thereof. Fractionated, refined or purified oils of these types can also be used. Specific examples of suitable long chain triglyceride-containing oils suitable for use in the oral compositions of the present invention include almond oil; babassu oil; borage oil; black currant seed oil; canola oil; castor oil; coconut oil; corn oil; cottonseed oil; emu oil; evening primrose oil; flax seed oil; grapeseed oil; groundnut oil; mustard seed oil; olive oil; palm oil; palm kernel oil; peanut oil; rapeseed oil; safflower oil; sesame oil; shark liver oil; soybean oil; sunflower oil; hydrogenated castor oil; hydrogenated coconut oil; hydrogenated palm oil; hydrogenated soybean oil; hydrogenated vegetable oil; a mixture of hydrogenated cottonseed oil and hydrogenated castor oil; partially hydrogenated soybean oil; a mixture of partially hydrogenated soybean oil and partially hydrogenated cottonseed oil; glyceryl trioleate; glyceryl trilinoleate; glyceryl trilinolenate; a Ω3-polyunsaturated fatty acid triglyceride containing oil; and mixtures thereof. The long chain triglyceride containing oils may be in particular selected from the group consisting of corn oil, olive oil, palm oil, peanut oil, safflower oil, sesame oil, soybean oil, castor oil, linseed oil, rape oil, rice bran oil, coconut oil, hydrogenated castor oil; partially hydrogenated soybean oil; glyceryl trioleate; glyceryl trilinoleate; a Ω3-polyunsaturated fatty acid triglyceride containing oil; and mixtures thereof.

Suitable saturated or unsaturated fatty alcohols have from about 6 to about 20 carbon atoms, cetearyl alcohol, lauryl alcohol, and mixtures thereof. For example, Lipowax (Cetearyl Alcohol and Ceteareth-20) are supplied and manufactured by Lipo Chemical.

General information on silicones including silicone fluids, gums and resins, as well as the manufacture of silicones, can be found in Encyclopedia of Polymer Science and Engineering, Volume 15, Second Edition, pp 204-308, John Wiley & Sons Inc. 1989 and Chemistry and Technology of Silicones, Walter Noll, Academic Press Inc, (Harcourt Brue Javanovich, Publishers, New York), 1968, pp 282-287 and 409-426.

The oral composition as disclosed herein may comprise additional ingredients which can be added optionally and which will be described below in further detail. Optionally, the present oral compositions may comprise some special ingredients. Optionally, the oral compositions of the present disclosure may comprise an emulsifier. Surprisingly, the multi-phase compositions preferably in the form of a water-in-oil emulsion can be formed even when no emulsifier is used. Thus, preferably, the multi-phase compositions preferably in the form of a water-in-oil emulsion may be formed even when no emulsifier is used. Without being bound by a theory it is believed that the low amount of aqueous phase, combined with the rheological properties, flow properties, drop melting point, and/or cone penetration consistency of the hydrophobic phase, and/or the process of preparation of the oral composition may help to disperse the aqueous phase into the hydrophobic phase and keep it dispersed without the use of an emulsifying agent. Thus, the present whitening oral compositions are preferably substantially free of an emulsifier. "Substantially free of an emulsifier" as understood herein means that the composition comprises less than 0.001% by weight of an emulsifier. More preferred the present whitening oral compositions are free of an emulsifier, i.e. do not comprise any emulsifier.

In addition, the oral composition may be also substantially free of acids and/or substantially free of alcohols, i.e. comprise less than 0.001% by weight of acids and/or alcohols, preferably do not comprise acids and/or alcohols. Without being bound by a theory it is believed that the decrease in surface tension produced by alcohol would decrease the retention time of the aqueous phase at the tooth surface, thereby decreasing the efficacy of the oral care actives. The presence of acids might contradict with the actives and/or may produce negative side effects as the tooth surface such as hypersensitivity etc.. Thus, the present oral compositions are preferably substantially free of acids, substantially free of alcohols, or substantially free of a mixture thereof.

Optionally, the hydrophobic phase of the oral composition may be substantially free of the bleaching agent(s).

In certain embodiments, the present oral compositions may comprise from 0.001% to 30% of an emulsifier. Any emulsifier may be used as long as the emulsifier chosen is non-toxic to the user. In one embodiment the emulsifier (or a combination of emulsifiers) favors the formation of a water in oil emulsion. In one embodiment the present oral compositions may comprise from about 0 to about 0.1%, or 0.1 to 5%, in another embodiment from 0.1 to 3%, and in another embodiment from 0.5% to 1.5% by weight of the oral composition, of emulsifier.

Classes of surfactants useful as emulsifiers include nonionic, anionic, cationic, amphoteric, synthetic emulsifying agents, and mixtures thereof. Many suitable nonionic and amphoteric surfactants are disclosed by U.S. Patent 3,988,433; U.S. Patent 4,051,234, and many suitable nonionic surfactants are disclosed by U.S. Patent 3,959,458.

In one embodiment, since water in oil emulsion are favored with more lipophilic emulsifiers, the emulsifier may have an HLB value of from about 1 to about 10, or from about 3 to about 8 or from about 4 to about 7, or from about 4 to about 6. Optionally, either a single emulsifier may be used or a combination of emulsifiers may be used, e.g. a blend of two or more emulsifiers such as a blend of two or more nonionic emulsifiers. In this regard an emulsifier that tends to form a water in oil emulsion and an emulsifier that forms an oil in water emulsion may be blended to achieve the requisite HLB for a water in oil emulsion. (HLB values are algebraically additive.)

Other emulsifiers optionally useful herein include natural emulsifying agents such as acacia, gelatin, lecithin and cholesterol; finely dispersed solids such as colloidal clays, bentonite, veegum (magnesium aluminum silicate; and synthetic emulsifying agents such as salts of fatty acids, sulfates such as sorbitan trioleate, sorbitan tristearate, sucrose distearate, propylene glycol monostearate, glycerol monostearate, propylene glycol monolaurate, sorbitan monostearate, sorbitan monolaurate, polyoxyethylene-4-lauryl ether, sodium lauryl sulfate, sulfonates such as dioctyl sosium sulfosuccinate, glyceryl esters, polyoxyethylene glycol esters and ethers, diethylene glycol monostearate, PEG 200 distearate, and sorbitan fatty acid esters, such as sorbitan monopalmitate, and their polyoxyethylene derivatives, polyoxyethylene glycol esters such as the monostearate, Polysorbate 80 (ethoxylated sorbitan monooleate) (supplied by Spectrum, etc.), and mixtures thereof.

An emulsifier may be a surfactant that is unreactive with the bleaching agent. For example, surfactants that are unreactive with the bleaching agent have no hydroxy groups, are free of nitrogen groups and linkages, are substantially free of metals such as Zn, etc.

The emulsifier may be a non-ionic surfactant. Nonionic surfactants include polyoxyethylene sorbitan fatty acid esters, e.g., materials sold under the trademark Tween. The number following the 'polyoxyethylene' part in the following section refers to the total number of oxyethylene $-(CH_2CH_2O)-$ groups found in the molecule. The number following the 'polysorbate' part is related to the type of fatty acid associated with the polyoxyethylene sorbitan part of the molecule. Monolaurate is indicated by 20, monopalmitate is indicated by 40, monostearate by 60, and monooleate by 80. Examples of such materials are polyoxyethylene (20) sorbitan monolaurate (Tween 20), polyoxyethylene (20) sorbitan monopalmitate (Tween 40), polyoxyethylene (20) sorbitan monostearate (Tween 60), polyoxyethylene (4) sorbitan monostearate (Tween 61), polyoxyethylene (20) sorbitan tristearate (Tween 65), polyoxyethylene (20) sorbitan monooleate (Tween 80), polyoxyethylene (5) sorbitan monooleate (Tween 81), and polyoxyethlene (20) sorbitan trioleate (Tween 85), and mixtures thereof. Polyoxyethylene fatty acid esters are also suitable and examples include those materials sold under the trademark Myrj such as polyoxyethylene (8) stearate (Myrj 45) and polyoxyethylene (40) stearate (Myrj 52), and mixtures thereof. Further nonionics include polyoxyethylene polyoxypropylene block polymers such as poloxamers and Pluronics.

Another suitable class of non-ionic surfactants for optional use in the present invention are polyoxyethylene fatty ethers, e.g., the materials sold under the trademark Brij. Examples of such materials are polyoxyethylene (4) lauryl ether (Brij 30), polyoxyethylene (23) lauryl ether (Brij 35), polyoxyethylene (2) cetyl ether (Brij 52), polyoxyethylene (10) cetyl ether (Brij 56), polyoxyethylene (20) cetyl ether (Brij 58), polyoxyethylene (2) stearyl ether (Brij 72), polyoxyethylene (10) stearyl ether (Brij 76), polyoxyethylene (20) stearyl ether (Brij 78), polyoxyethylne (2) oleyl ether (Brij 93), polyoxyethylene (10) oleyl ether, and polyoxyethylene (20) oleyl ether (Brij 99), and mixtures thereof.

In one embodiment of the invention, a portion of a non-ionic surfactant may be substituted with a lipophilic surfactant, e.g., sorbitan fatty acid esters such as the materials sold under the trademark Arlacel. Suitable lipophilic surfactants include sorbitan monolaurate (Arlacel 20), sorbitan monopalmitate (Arlacel 40), sorbitan monostearate (Aracel 60), sorbitan monooleate (Arlacel 80), sorbitan sesquioleate (Arlacel 83), and sorbitan trioleate (Arlacel 85), and mixtures thereof. Typically, from about 2% to about 90% of the level of the nonionic surfactant may be substituted by a lipophilic surfactant, in another embodiment from about 25% to about 50%. In one embodiment the emulsifier may be Aerosol OT (sodium dioctyl sulfosuccinate) manufactured by Cytec.

Additional, ingredients of the oral composition

Thickening Agents, Viscosity Modifiers, and/or Particulate Fillers

The oral compositions herein optionally comprise a safe and effective amount of a thickening agent, viscosity modifier and/or particulate fillers. A thickening agent may further provide acceptable rheology of the oral composition. A viscosity modifier may further function to inhibit settling and separation of components or control settling in a manner that facilitates re-dispersion and may control flow properties of the oral composition. In addition, a thickening agent or viscosity modifier may facilitate use of the present oral compositions with suitable applications devices, such as strip, films or dental trays by increasing the retention onto the surfaces of the application devices. A thickening agent herein may also serve as an adhesive.

When optionally present, a thickening agent, viscosity modifier, and/or particulate filler may be present at a level of from about 0.01% to about 99%, in particular from about 0.1% to about 50%, more particular from about 1% to about 25%, and even more particular from about 1% to about 10%, by weight of the oral composition.

Suitable thickening agents, viscosity modifiers, and/or particulate fillers that can be used herein include organo modified clays, silicas, synthetic polymers such as crosslinked siloxanes, cellulose derivatives (e.g. methylcellulose, carboxymethylcellulose, hydroxyethylcellulose, hydroxypropylcellulose, hydroxy-propylmethylcellulose, etc.), carbomer polymers (e.g. crosslinked polyacrylic acid copolymer or homopolymer and copolymers of acrylic acid cross linked with a polyalkenyl polyether),polymethacryates, natural and synthetic gums, karaya gum, guar gum, gelatin, algin, sodium alginate, tragacanth, chitosan, polyethylene, polyethylne oxide, acrylamide polymers, polyacrylic acid, polyvinyl alcohol, polyamines, polyquartemary compounds, ethylene oxide polymers, polyvinylpyrrolidone, cationic polyacrylamide polymers, wax, including paraffin wax or microcrystalline waxes, polyethylene, their derivatives, fumed silica, and mixtures thereof, olefin copolymers, hydrogenated styrene-diene copolymers, styrene polyesters, rubber, polyvinylchloride, nylon, fluorocarbon, polyurethane prepolymer, polystyrene, polypropylene, cellulosic resins, acrylic resins, elastomers, poly(n-butyl vinyl ether), poly(styrene-co-maleic anhydride), poly(alkyl fumarate co-vinyl acetate), alkylated polystyrene, poly(t-butyl styrene), or mixtures thereof.

Examples of polyethylene include A-C 1702 or A-C 6702 made by Honeywell Corp. (Morristown, NJ), with a penetration value of about 98.5 and about 90.0, respectively, under ASTM D-1321; polyethylene Performalene series from Baker Hughes; this includes polyethylene Performalene 400 from Baker Hughes Inc. (Houston, TX). Examples of microcrystalline wax include the Multiwax series from Sonneborn (Parsippany, NJ Crompton (Witco); these include Multiwax 835, Multiwax 440, Multiwax 180, and mixtures thereof.

Examples of polymethacyrlates include, for example, polyacrylate-co-methacrylate, polymethacrylate-co-styrene, or combinations thereof. Examples of elastomers include, for example, hydrogenated styrene-co-butadiene, hydrogenated styrene-co-isoprene, ethylene-ethylene-propylene polymer, ethylene-propylene polymer, styrene-ethylene-ethylene-propylene-styrene polymer or combinations thereof. An example of a rubber includes hydrogenated polyisoprene. Other examples of viscosity modifiers can be found in "Chemistry and Technology of Lubricants," Chapman and Hall (2nd Ed. 1997).

Suitable example carbomers comprises the class of homopolymers of acrylic acid crosslinked with an alkyl ether of pentaerythritol or an alkyl ether of sucrose. Carbomers are commercially available from B.F. Goodrich as the Carbopol® series, such as Carbopol 934, 940, 941, 956, and mixtures thereof. Homopolymers of polyacrylic acid are described, for example, in U.S. Pat. No. 2,798,053. Other examples of homopolymers which are useful include Ultrez 10, ETD 2050, and 974P polymers, which are available from The B.F.Goodrich Company (Greenville, SC). Such polymers are homopolymers of unsaturated, polymerizable carboxylic monomers such as acrylic acid, methacrylic acid, maleic acid, itaconic acid, maleic anhydride, and the like.

Optional additional Oral Care Active Agents

The oral composition of the present invention may optionally comprise a safe and effective amount of an additional oral care active agent such as any material that is generally considered safe for use in the oral cavity and that provides changes to the overall appearance and/or health of the oral cavity. Suitable additional oral care actives include one or more from anticalculus agent(s), fluoride ion source, antimicrobial agent(s), dentinal desensitizing agent(s), anesthetic agent(s), antifungal agent(s), anti-inflammatory agent(s), selective H-2 antagonist(s), anticaries agent(s), nutrient(s), and mixtures thereof. The additional oral care active agent may contain an active at a level where upon directed use, the benefit sought by the wearer is promoted without detriment to the oral surface to which it is applied. Examples of the oral conditions these actives address include, but, are not limited to, appearance and structural changes to teeth, stain removal, plaque removal, tartar removal, cavity prevention and treatment, inflamed and/or bleeding gums, mucosal wounds, lesions, ulcers, aphthous ulcers, cold sores, tooth abscesses, and the elimination of mouth malodor resulting from the conditions above and other causes such as microbial proliferation. The level of the additional oral care active in the oral compositions is generally, unless specifically noted, from about 0.01% to about 50%, in particular from about 0.1% to about 20%, more particular from about 0.5% to about 10%, and more particular from about 1% to about 7%, by weight of the oral composition.

As the present oral composition is directed to bleaching the tooth surface and removing or decreasing the stain attached thereto, it may be preferred to add a safe and effective amount of at least one anticalculus agent to the oral compositions as disclosed herein. Said amount is generally from about 0.01% to about 40% by weight in particular from about 0.1% to about 25%, more particular from about 4.5% to about 20%, and more particular from about 5% to about 15%, by weight of the oral composition. The anticalculus agent may also be compatible with the other components of the oral composition, in particular the whitening agent. The anticalculus agent may be selected from the group consisting of polyphosphates and salts thereof; polyamino propane sulfonic acid (AMPS) and salts thereof; polyolefin sulfonates and salts thereof; polyvinyl phosphates and salts thereof; polyolefin phosphates and salts thereof; diphosphonates and salts thereof; phosphonoalkane carboxylic acid and salts thereof; polyphosphonates and salts thereof; polyvinyl phosphonates and salts thereof; polyolefin phosphonates and salts thereof; polypeptides; and mixtures thereof, wherein the mentioned salts are usually alkali metal salts. If present, the anticalculus agent is preferably selected from the group consisting of polyphosphates and salts thereof; diphosphonates and salts thereof; and mixtures thereof, more preferred selected from the group consisting of pyrophosphate, polyphosphate, and mixtures thereof. In particular preferred are anticalculus agents in the present oral composition which also show a stabilizing effect to the bleaching agents, such as pyrophosphates, polyphosphates, polyphophonates and mixtures thereof.

For example the anticalculus agent may be a polyphosphate. A polyphosphate is generally understood to comprise of two or more phosphate molecules arranged primarily in a linear configuration, although some cyclic derivatives may be present. Linear polyphosphates correspond to $(X PO_3)_n$ where n is about 2 to about 125, wherein preferably n is greater than 4, and X is for example sodium, potassium, etc. For $(X PO_3)_n$ when n is at least 3 the polyphosphates are glassy in character. Counter-ions for these phosphates may be the alkali metal, alkaline earth metal, ammonium, $C_2$-$C_6$ alkanolammonium and salt mixtures. Polyphosphates are generally employed as their wholly or partially neutralized water soluble alkali metal salts such as potassium, sodium, ammonium salts, and mixtures thereof. The inorganic polyphosphate salts include alkali metal (e.g. sodium) tripolyphosphate, tetrapolyphosphate, dialkyl metal (e.g. disodium) diacid, trialkyl metal (e.g. trisodium) monoacid, potassium hydrogen phosphate, sodium hydrogen phosphate, and alkali metal (e.g. sodium) hexametaphosphate, and mixtures thereof. Polyphosphates larger than tetrapolyphosphate usually occur as amorphous glassy materials, such as those manufactured by FMC Corporation which are commercially known as Sodaphos (n≈6), Hexaphos (n≈13), Glass H (n≈21), and mixtures thereof. If present, the present oral compositions will typically comprise from about 0.5% to about 20%, in particular from about 4% to about 15%, more particular from about 6% to about 12%, by weight of the oral composition of polyphosphate.

The pyrophosphate salts useful in the present oral compositions include, alkali metal pyrophosphates, di-, tri-, and mono-potassium or sodium pyrophosphates, dialkali metal pyrophosphate salts, tetraalkali metal pyrophosphate salts, and mixtures thereof. For example, the pyrophosphate salt is selected from the group consisting of trisodium pyrophosphate, disodium dihydrogen pyrophosphate ($Na_2H_2P_2O_7$), dipotassium pyrophosphate, tetrasodium pyrophosphate ($Na_4P_2O_7$), tetrapotassium pyrophosphate ($K_4P_2O_7$), and mixtures thereof, wherein tetrasodium pyrophosphate is preferred. Tetrasodium pyrophosphate may be the anhydrous salt form or the decahydrate form, or any other species stable in solid form in the present oral compositions. The salt is in its solid particle form, which may be its crystalline and/or amorphous state, with the particle size of the salt preferably being small enough to be aesthetically acceptable and readily soluble during use. The level of pyrophosphate salt in the present oral compositions may be from about 1.5% to about 15%, in particular from about 2% to about 10%, and more particular from about 3% to about 8%, by weight of the oral composition.

The phosphate sources, including but are not limited to, polyphosphates and pyrophosphates, are described in more detail in Kirk & Othmer, Encyclopedia of Chemical Technology, Fourth Edition, Volume 18, Wiley-Interscience Publishers (1996), pages 685-707, incorporated herein by reference in its entirety, including all references incorporated into Kirk & Othmer.

Polyolefin phosphonates include those wherein the olefin group contains 2 or more carbon atoms. Polyvinylphosphonates include polyvinylphosphonic acid. Diphosphonates and salts thereof include azocycloalkane-2,2-diphosphonic acids and salts thereof, ions of azocycloalkane-2,2-diphosphonic acids and salts thereof (such as those which the alkane moiety has five, six or seven carbon atoms, in which the nitrogen atom is unsubstituted or carries a lower alkyl substitutent, e.g. methyl), azocyclohexane-2,2-diphosphonic acid, azocyclopentane-2,2-diphosphonic acid, N-methyl-azocyclopentane-2,3-diphosphonic acid, EHDP (ethanehydroxy-1,1,-diphosphonic acid), AHP (azacycloheptane-2,2-diphosphonic acid, a.k.a. 1-azocycloheptylidene-2,2-diphosphonic acid), ethane-1-amino-1,1-diphosphonate, dichloromethane-diphosphonate, etc. Phosphonoalkane carboxylic acid or their alkali metal salts include PPTA (phosphonopropane tricarboxylic acid), PBTA (phosphonobutane-1,2,4-tricarboxylic acid), each as acid or alkali metal salts.

In addition or alternatively, antimicrobial antiplaque agents may also be optionally present in the present oral compositions. Such agents may include, but are not limited to, triclosan, 5-chloro-2-(2,4-dichlorophenoxy)-phenol, as described in The Merck Index, 11th ed. (1989), pp. 1529 (entry no. 9573) in U.S. Patent No. 3,506,720, and in European Patent Application No. 0,251,591; chlorhexidine (Merck Index, no. 2090), alexidine (Merck Index, no. 222; hexetidine (Merck Index, no. 4624); sanguinarine (Merck Index, no. 8320); benzalkonium chloride (Merck Index, no. 1066); salicylanilide (Merck Index, no. 8299); domiphen bromide (Merck Index, no. 3411); cetylpyridinium chloride (CPC) (Merck Index, no. 2024; tetradecylpyridinium chloride (TPC); N-tetradecyl-4-ethylpyridinium chloride (TDEPC); octenidine; delmopinol, octapinol, and other piperidino derivatives; In addition or alternatively present may be effective antimicrobial amounts of essential oils and combinations thereof for example citral, geranial, and combinations of menthol, eucalyptol, thymol and methyl salicylate; antimicrobial metals and salts thereof for example those providing zinc ions, stannous ions, copper ions, and/or mixtures thereof; bisbiguanides, or phenolics; antibiotics such as augmentin, amoxicillin, tetracycline, doxycycline, minocycline, and metronidazole; and analogs and salts of the above antimicrobial antiplaque agents and/or anti-fungals such as those for the treatment of candida albicans. If present, these agents generally are present in a safe and effective amount for example from about 0.1% to about 5% by weight of the present oral compositions.

In addition or alternatively, the present oral composition may comprise a safe and effective amount of an anticaries agent, and mixtures thereof. The anticaries agent may be selected from the group consisting of xylitol, fluoride ion source providing free fluoride ions, and mixtures thereof. A suitable fluoride ion source may be selected from the group consisting of sodium fluoride, stannous fluoride, indium fluoride, organic fluorides such as amine fluorides, and sodium monofluorophosphate, wherein sodium fluoride is preferred. Preferably the instant oral compositions provide from about 50 ppm to 10,000 ppm, more preferably from about 100 to 3000 ppm, of fluoride ions in the oral compositions that contact dental surfaces when used with the oral composition as disclosed herein.

In addition or alternatively, coolants, desensitizing agents and numbing agents can be used as optional ingredients in oral compositions of the present invention, in particular at a level of from about 0.001% to about 10%, more particular from about 0.1% to about 1%, by weight of the oral composition. Coolants, desensitizing agents and numbing agents may decrease potential negative perceptions, such as tingling, burning etc.. Coolant can be any of a wide variety of materials. Included among such materials are carboxamides, menthol, ketals, diols, and mixtures thereof. Optional coolants in the present oral compositions may be the paramenthan carboxyamide agents such as N-ethyl-p-menthan-3-carboxamide (known as "WS-3"), N,2,3-trimethyl-2-isopropylbutanamide (known as "WS-23"), menthol, 3-1-menthoxypropane-1,2-diol (known as TK-10), menthone glycerol acetal (known as MGA) menthyl lactate (known as Frescolat®), and mixtures thereof. The terms menthol and menthyl as used herein include dextro- and levorotatory isomers of these compounds and racemic mixtures thereof. Desensitizing or Anti-pain agent may include, but are not limited to, strontium chloride, potassium nitrate, natural herbs such as gall nut, Asarum, Cubebin, Galanga, scutellaria, Liangmianzhen, Baizhi, etc.. Suitable numbing agents include benzocaine, lidocaine, clove bud oil, and ethanol.

In addition or alternatively, anti-inflammatory agents may be present in the oral compositions as disclosed herein. Such agents may include, but are not limited to, non-steroidal anti-inflammatory agents such as aspirin, ketorolac, flurbiprofen, ibuprofen, naproxen, indomethacin, aspirin, ketoprofen, piroxicam and meclofenamic acid, COX-2 inhibitors such as valdecoxib, celecoxib and rofecoxib, and mixtures thereof. If present, the anti-inflammatory agents generally comprise from about 0.001% to about 5% by weight of the oral compositions.

In addition or alternatively, nutrients, such as minerals, may improve the teeth and the tooth surface and thus can be included with the oral compositions as disclosed herein. Suitable minerals are e.g. calcium, phosphorus, fluoride, zinc, manganese, potassium and mixtures thereof. These minerals are e.g disclosed in Drug Facts and Comparisons (loose leaf drug information service), Wolters Kluer Company, St. Louis, Mo., ©1997, pp10-17.

In addition or alternatively, the oral compositions as disclosed herein may optionally comprise a safe and effective amount of a flavoring agent. Suitable flavoring agents include oil of wintergreen, oil of peppermint, oil of spearmint, clove bud oil, menthol, anethole, methyl salicylate, eucalyptol, 1-menthyl acetate, sage, eugenol, parsley oil, oxanone, alpha-irisone, marjoram, lemon, orange, propenyl guaethol, cinnamon, vanillin, thymol, linalool, cinnamaldehyde glycerol acetal (known as CGA), and mixtures thereof. If present the flavoring agents are generally used at levels of from about 0.01% to about 30%, in particular from about 1% to about 20%, more particular from about 1.5% to about 15%, by weight of the oral composition.

In addition or alternatively, the present oral compositions may optionally comprise sweetening agents including sucralose, sucrose, glucose, saccharin, dextrose, levulose, lactose, mannitol, sorbitol, fructose, maltose, xylitol, saccharin salts, thaumatin, aspartame, D-tryptophan, dihydrochalcones, acesulfame and cyclamate salts, especially sodium cyclamate and sodium saccharin, and mixtures thereof. If present, the oral composition contains from about 0.1% to about 10% of these agents, in particular from about 0.1% to about 1%, by weight of the oral composition.

In addition or alternatively, dyes, pigments, colorants, and mixtures thereof may optionally be included in the present oral composition to give the oral compositions herein colored appearance. An advantage of adding pigments and/or colorants to the oral compositions herein is that it will allow the user to see if the oral composition covers their teeth evenly and completely, since coverage is easier to see with a colored oral composition. In addition or alternatively, the colorant may provide color similar to the color of bleached teeth. Colorants useful herein are stable with the bleach agent and are those recognized as safe. The levels of dye, pigments and colorants that are optionally used herein are in the range of about 0.05% to about 20%, in particular from about 0.10% to about 15% and more particular from about 0.25% to about 5% by weight of the oral composition.

Bleaching Efficacy

The bleaching efficacy of the present invention as measured per the clinical protocol as disclosed herein and calculated as -Δb* may be at least about 0.25, preferably at least about 0.5, more preferred at least about 1.0, even more preferred at least about 1.5 . Generally, a change in yellowness as measured per the clinical protocol as disclosed herein and calculated as -Δb* of at least 0.25 is noticeable.

The ratio of bleaching efficacy of the present invention as measured per the clinical protocol as disclosed herein and calculated as -Δb* to the net weight percent of bleaching agent present in the overall oral composition may be at least about 2.5, preferably at least about 5, more preferred at least about 10, even more preferred at least about 15.

The bleaching efficacy of the present invention as measured per the clinical protocol as disclosed herein and calculated as -Δb* may be at least about 10%, preferably at least about 100%, more preferred at least about 1000%, even more preferred at least about 10,000% more than the bleaching efficacy of a comparative oral composition in form of an aqueous solution or aqueous gel. The comparative oral composition comprises the same bleaching agent at the same overall concentration dissolved into the aqueous solution or aqueous gel.

CLINICAL PROTOCOL

The bleaching efficacies of the oral compositions are measured using the following clinical protocol. Per treatment group, 17 to 25 subjects are recruited to complete the clinical study. Recruited subjects must have four natural maxillary incisors with all measurable facial sites. The mean baseline L* of the group of subjects must be from 71 to 76, and the mean baseline b* of the group of subjects must be from 13 to 18. In addition, subjects with malocclusion on maxillary anterior teeth, severe or atypical intrinsic staining, such as that caused by tetracycline, fluorosis or hypocalcification, dental crowns or restorations on the facial surfaces of maxillary anterior teeth, self-reported medical history of melanoma, current smoking or tobacco use, light-sensitivity or a pigmentation skin disorder, self-reported tooth sensitivity, or previous tooth whitening using a professional treatment, over-the-counter kit, or investigational product, are excluded from the study. Subjects are provided with take-home kits with Crest Cavity Protection toothpaste and Oral-B Indicator soft manual toothbrush (both from Procter & Gamble, Cincinnati, OH, USA) to be used twice a day in the customary manner.

The subjects use a toothbrush ("Anchor 41 tuft white toothbrush" from Team Technologies, Inc. Morristown, TN, USA) to brush their teeth with water for 30 seconds prior to being treated with the oral composition. The maxillary anterior teeth of each subject are treated with the oral composition for 60 minutes once daily using a strip of polyethylene as a delivery carrier. The polyethylene strips are 66mm x 15mm in size and 0.0178mm thick. 0.6 grams to 0.8 grams of the oral composition are applied across each strip of polyethylene prior to applying to the maxillary anterior teeth.

If the oral composition is used with electromagnetic radiation:

1) After 50 minutes of treatment with the oral composition on the strip, the electromagnetic radiation is applied toward the facial surfaces of the maxillary anterior teeth for 10 minutes,

2) The electromagnetic radiation is directed toward the maxillary anterior teeth through the strip and through the oral composition,

3) The strip needs to allow at least about 90% of the electromagnetic radiation from 400 nm to 500 nm to pass through, and

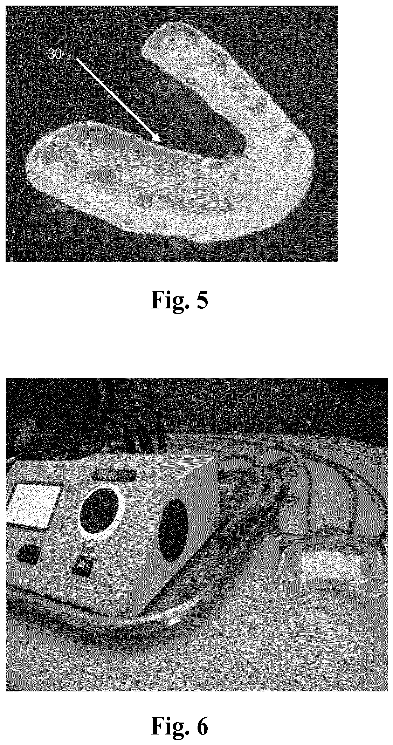

4) The electromagnetic radiation is delivered via four fiber-optic cables (model number M71L01 from Thorlabs, Newton, NJ, USA) connected to four high power LEDs with a peak intensity wavelength of 455nm (model number M455F1 from Thorlabs, Newton, NJ, USA) as shown in Fig. 6. The four LEDs are run at 1000mA each using an LED Driver and Hub (model numbers DC4104 and DC4100-HUB from Thorlabs, Newton, NJ, USA). The exit ends of the four fiber-optic cables are mounted behind a transparent mouthpiece to help position the electromagnetic radiation reproducibly against the outer surface of the strip. The exit ends of the four fiber-optic cables are about 7mm away from the exit surface of the mouthpiece with the electromagnetic radiation passing through the transparent mouthpiece. The bite-shelf of the mouthpiece is offset such that the transparent window through which the electromagnetic radiation passes toward the maxillary anterior teeth is 7.4 mm high. Also, the transparent window through which the electromagnetic radiation passes toward the maxillary anterior teeth is 40mm long measured linearly from end to end (not including the curvature). The exit ends of the fiber-optic cables are positioned & angled such that the cones of electromagnetic radiation exiting from the fiber-optic cables are centered within the transparent window through which the electromagnetic radiation passes toward the maxillary anterior teeth as shown in Figure 6. Also, the exit ends of the four fiber-optic cables are spaced such that the cones of electromagnetic radiation are spaced across the length of the transparent window through which the electromagnetic radiation passes toward the maxillary anterior teeth as shown in Figure 6. The intensity of the electromagnetic radiation from 400 nm to 500 nm measured at the central axis of each cone of electromagnetic radiation exiting at the exit surface of the transparent window through which the electromagnetic radiation passes toward the maxillary anterior teeth needs to be about 205 mW/cm2 as measured by the method disclosed herein.

Once 60 minutes of the treatment with the oral composition is completed, the strip is removed. This treatment is applied once daily for a minimum of 7 days.

The change in tooth color due to the treatment with the oral composition is measured using the procedure described below the day after the 7th treatment.

Tooth color is measured using a digital camera having a lens equipped with a polarizer filter (Camera model no. CANON EOS 70D from Canon Inc., Melville, NY with NIKON 55mm micro-NIKKOR lens with adapter). The light system is provided by Dedo lights (model number DLH2) equipped with 150 watt, 24V bulbs model number (Xenophot model number HL X64640), positioned about 30 cm apart (measured from the center of the external circular surface of one of the glass lens through which the light exits to the other) and aimed at a 45 degree angle such that the light paths intersect at the vertical plane of the chin rest about 36 cm in front of the focal plane of the camera. Each light has a polarizing filter (Lee 201 filter), and a cutoff filter (Rosco 7 mil Thermashield filter from Rosco, Stamford, CT, USA).

At the intersection of the light paths, a fixed chin rest is mounted for reproducible repositioning in the light field. The camera is placed between the two lights such that it's focal plane is about 36 cm from the vertical plane of the chin rest. Prior to beginning the measurement of tooth color, color standards are imaged to establish calibration set-points. A Munsell N8 grey standard is imaged first. The white balance of the camera is adjusted such that the RGB values of grey are 200. Color standards are imaged to get standard RGB values of the color chips. The color standards and grey standard are listed below (from Munsell Color, Division of X-rite, Grand Rapids, MI, USA). Each color standard is labeled with the Munsell nomenclature. To create a grid of color standards, they can be arranged in the following manner. This enables multiple color standards to be contained in a single image captured of the grid of color standards.

Color standard grid 1

| 7.5R 6 8 | 2.5R 6 10 | 10YR 6.5 3 | Polarization check | 5R 7 8 | N 3.5 0 |
|---|---|---|---|---|---|
| 7.5RP 6 6 | 10R 5 8 | 5YR 7 3 | 2.5Y 8.5 2 | 2.2YR 6.47 4.1 | 7.5YR 7 4 |
| 5YR 8 2 | N 8 0 | 10R 7 4 | N 8 0 | 5YR 7.5 2.5 | 2.5Y 8 4 |
| 5YR 7 3.5 | 5YR 7 2.5 | 5YR 5 2 | 5YR 7.5 2 | N 6.5 0 | N 9.5 0 |

Color standard grid 2

| 5YR 7.5 3.5 | 2.5Y 6 4 | 10YR 7.5 3.5 | 2.5R 7 8 | 7.5R 7 8 | 10YR 7.5 2 |
|---|---|---|---|---|---|
| 10YR 7.5 2.5 | N 5 0 | 2.5R 6 8 | 10YR 7 2 | 5R 7 4 | 10YR 7 2.5 |
| N 6.5 0 | 7.5RP 6 8 | 7.5R 8 4 | 5Y 8 1 | 7.5YR 8 2 | 2.2YR 6.47 4.1 |
| N 5 0 | 2.5Y 8 4 | 10YR 7 3 | N 9.5 0 | 10RP 7 4 | 2.5Y 7 2 |

Color standard grid 3

| 5R 6 10 | N 8.5 0 | 10YR 6.5 3.5 | 10RP 6 10 | N 8 0 | 7.5YR 7 3 |
|---------|---------|--------------|-----------|-------|-----------|
| 2.5Y 3.5 0 | 10YR 7 3.5 | 5Y 8.5 1 | 5YR 8 2.5 | 5YR 7.5 3 | 5R 5 6 |
| 10YR 7.5 3 | 5YR 6.5 3.5 | 2.5YR 5 4 | 2.5Y 8 2 | 10YR 8 2 | 2.5Y 7 2 |
| 2.5R 6 6 | 5R 7 6 | 10YR 8 2.5 | 10R 5 6 | N 6.5 0 | 7.5YR 8 3 |

For baseline tooth color, subjects use a toothbrush ("Anchor 41 tuft white toothbrush" from Team Technologies, Inc. Morristown, TN, USA) to brush their teeth with water to remove debris from their teeth. Each subject then uses cheek retractors (from Washington Scientific Camera Company, Sumner, WA, USA; treated with at frosted matte finish at A&B Deburring Company, Cincinnati, OH, USA) to pull the cheeks back and allow the facial surfaces of their teeth to be illuminated. Each participant is instructed to bite their teeth together such that the incisal edges of the maxillary incisors contact the incisal edges of the mandibular incisors. The subjects are then positioned on the chin rest at the intersection of the light paths in the center of the camera view and the tooth images are captured. After all subjects are imaged, the images are processed using image analysis software (Optimas manufactured by Media Cybernetics, Inc. of Silver Spring, MD). The central four incisors are isolated and the average RGB values of the teeth are extracted.

After the subjects have used a whitening product but prior to capturing participant's tooth images, the system is set to the baseline configuration and calibrated as previously discussed. After calibration, each participant is imaged a second time using the same procedure as before making sure the subject is in the same physical position as the pretreatment image including orientation of the teeth. The images are processed using the image analysis software to obtain the average RGB values of the central four maxillary incisors. The RGB values of all of the images are then mapped into CIE L*'a*b* color space using the RGB values and the L*a*b* values of the color chips on the color standard. The L*a*b* values of the color chips on the color standard are measured using a Photo Research SpectraScan PR650 from Photo Research Inc., LA using the same lighting conditions described for capturing digital images of the facial dentition. The PR650 is positioned the same distance from the color standards as the camera. Each chip is individually measured for L*a*b* after calibration according to the manufacturer's instructions. The RGB values are then transformed into L*a*b* values using regression equations such as:

$$L^* = \ 25.16 + 12.02*(R/100) + 11.75*(G/100) - 2.75*(B/100) + 1.95*(G/100)^3$$

$$a^* = \ -2.65 + 59.22*(R/100) - 50.52*(G/100) + 0.20*(B/100) - 29.87*(R/100)^2$$
$$+ 20.73*(G/100)^2 + 8.14*(R/100)^3 - 9.17(G/100)^3 + 3.64*[(B/100)^2]*[R/100]$$

$$b^* = \ -0.70 + 37.04*(R/100) + 12.65*(G/100) - 53.81*(B/100) - 18.14*(R/100)^2$$
$$+ 23.16*(G/100)*(B/100) + 4.70*(R/100)^3 - 6.45*(B/100)^3$$

The $R^2$ for L*, a*, and b* must be > 0.95. Each study should have its own equations.

These equations are generally valid transformations in the area of tooth color (60 < L* < 95, 0 < a* < 14, 6 < b* < 25). The data from each participant's set of images is then used to calculate product whitening performance in terms of changes in L*, a* and b* -a standard method used for assessing whitening benefits. Changes in L* is defined as $\Delta L^* = L^*_{\text{day after 7 treatments}} - L^*_{\text{baseline}}$. A positive change indicates improvement in brightness. Changes in a* (red-green balance) is defined as $\Delta a^* = a^*_{\text{day after 7 treatments}} - a^*_{\text{baseline}}$. A negative change indicates teeth which are less red. Changes in b* (yellow-blue balance) is defined as $\Delta b^* = b^*_{\text{day after 7 treatments}} - b^*_{\text{baseline}}$. A negative change indicates teeth are becoming less yellow. $-\Delta b^*$ is used as the primary measure of bleaching efficacy. The overall color change is calculated by the equation $\Delta E = (\Delta L^{*2} + \Delta a^{*2} + \Delta b^{*2})^{1/2}$.

After using the whitening products, color changes in CIE Lab color space can be calculated for each participant based on the equations given.

To validate the above clinical protocol, the bleaching efficacy (calculated as $-\Delta b^*$) of Example-IA (delivered on a strip and used with electromagnetic radiation as disclosed herein) needs to be measured the day after the 7th treatment and demonstrated to be > 0.5.

Application Systems

According to the present invention a delivery system is used for delivering the present oral compositions to the tooth surface. The present invention includes a delivery system comprising the present oral compositions in combination with a delivery carrier. For example, the delivery system may comprise a first layer of a carrier material and a second layer comprising the oral composition described herein, whereby the bleaching agent is releasably located within the present oral composition. A suitable first layer may comprise a delivery carrier including a strip of material, a dental tray, a sponge material, and mixtures thereof. Suitable strips of material or permanently deformable strips are for example disclosed in U.S. Pat. Nos; 6,136,297; 6,096,328; 5,894,017; 5,891,453; and 5,879,691, all to Sagel, et al., and all assigned to The Procter & Gamble Company, and in U.S. Pat. Nos. 5,989,569 and 6,045,811 both to Dirksing, et al., and both assigned to The Procter & Gamble Company; and in patent application US 2014/0178443 A1.

The delivery carrier may be attached to the teeth via an attachment means that is part of the delivery carrier, for example the delivery carrier may optionally be of sufficient size that, once applied, the delivery carrier overlaps with the oral soft tissues rendering more of the teeth surface available for bleaching. The delivery carrier may also be attached to the oral cavity by physical interference or mechanical inter-locking between the delivery carrier and the oral surfaces including the teeth.

The delivery carrier may be transparent or translucent to electromagnetic radiation with wavelengths from about 200 nm to about 1700 nm. Preferably, the delivery carrier is transparent or translucent to electromagnetic radiation with wavelengths from about 400nm to about 500nm. Preferably, the delivery carrier allows from about 10, 20, or 30 % to about 40, 50, 60, 70, 80, 90, or 100% of electromagnetic radiation from about 400 nm to about 500 nm to pass through.

Where the delivery carrier is a strip of material, the second layer composition may be coated on the strip, or be applied by the user to the strip, or be applied by the user to the teeth and then the strip may be placed over the coated teeth. The amount of oral composition applied to the strip or teeth may depend upon the size and capacity of the strip, concentration of the active and the desired benefit. From about 0.0001, 0.001 or 0.01 grams to about 0.01, 0.1, 1, or 5 grams of oral composition may be used or any other numerical range which is narrower and which falls within such broader numerical range, as if such narrower numerical ranges were all expressly written herein, in particular from about 0.001g to about 0.5g and more particular from about 0.1g to about 0.4g of oral composition may be used. In addition or alternatively, from about 0.0001, 0.001 or 0.01 grams to about 0.01, 0.1, 0.5, or 1 grams oral composition per square centimeter of material ($g/cm^2$) may be used or any other numerical range which is narrower and which falls within such broader numerical range, as if such narrower numerical ranges were all expressly written herein, in particular less than about 0.2 $g/cm^2$, more particular from about 0.0001$g/cm^2$ to about 0.1 $g/cm^2$, and even more particular from about 0.01 $g/cm^2$ to about 0.04 $g/cm^2$. In addition or alternatively, from about 1 microgram to about 5000 micrograms bleaching agent per square centimeter of material (microgram/cm2), preferably from about 10 micrograms/$cm^2$ to about 500 micrograms/$cm^2$, and more preferably from about 50 micrograms/$cm^2$ to about 100 micrograms/$cm^2$ bleaching agent per square centimeter of material may be used.

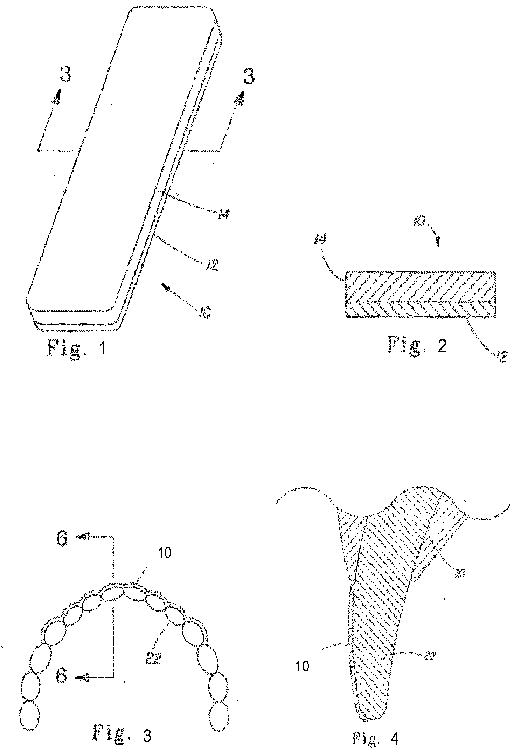

Referring now to the drawings, and more particularly to Fig. 1, there is shown a first embodiment of a suitable delivery system 10, representing a delivery system for delivering bleach actives provided by an oral composition as disclosed herein to the teeth and the oral cavity. Delivery system 10 comprises a material in strip form 12 of material which is substantially flat, preferably with rounded corners. Onto said strip 12 a second layer 14 comprising the present oral composition is releasably applied. The second layer 14 may be homogenous and may be uniformly and continuously coated onto strip 12, as shown in the cross-sectional view of Fig. 2. In addition or alternatively, the second layer 14 comprising the present oral compositions may be a continuous coating only along a longitudinal axis of a portion of strip of material 12 or may be applied as stripes, spots, and/or other patterns.

However, in certain embodiments the second layer 14 may be a laminate or separated layers of components, an amorphous mixture of components, separate stripes or spots or other patterns of different components, or a combination of these structures, including a continuous coating of the second layer 14 along a longitudinal axis of a portion of the strip of material 12. In addition or alternatively, the second layer 14 may contain or is itself an active, such as a composition, compound, or mixture capable of influencing or effecting a desired change in appearance or structure of the surface it contacts. As discussed previously, example actives include: hydrogen peroxide, carbamide peroxide, sodium fluoride, sodium monofluorophosphate, pyrophosphate, chlorhexidine, polyphosphate, triclosan, and enzymes. Examples of appearance and structural changes include, but are not necessarily limited to: whitening, stain bleaching, stain removal, remineralization to form fluorapatite, plaque removal, and tartar removal.

In addition the second layer 14 oral composition may comprise adhesive means in order to stably attach the delivery system 10 to the tooth surface. Alternatively, the oral composition as disclosed herein may provide the intended stickiness and adhesiveness by its own, for example by choosing a hydrophobic phase which already provides adhesive properties and/or by adding adhesive material to the oral compositions of the present invention. If added, an adhesive will be preferred which provides additional properties, such as thickening/rheology modifying properties.

Figs 3 and 4 show a delivery system 10 of the present invention applied to the tooth surface of a plurality of adjacent teeth. Embedded in adjacent soft tissue 20 is a plurality of adjacent teeth 22. Adjacent soft tissue 20 herein defined as soft tissue surfaces surrounding the tooth structure including: papilla, marginal gingival, gingival sulculus, inter dental gingival, and gingival gum structure on lingual and buccal surfaces up to and including muco-gingival junction on the pallet.

In both Figs. 3 and 4, delivery system 10 represents a strip 12 and second layer 14 comprising the present composition, wherein the second layer 14 is located on the side of strip of material 12 facing teeth 22. Oral composition of second layer 14 may be pre-applied to strip of material 12, or may be applied to strip of material 12 by the user prior to application to the teeth. Alternatively, the oral composition of second layer 14 may be applied directly to teeth 22 by the user and then covered by a strip 12. In any case, strip of material 12 has a thickness and flexural stiffness such that it can conform to the contoured surfaces of teeth 22 and to adjacent soft tissue 20. Thus, the strip of material 12 has sufficient flexibility to form to the contours of the oral surface, the surface being a plurality of adjacent teeth 22. The strip 12 may also readily conformable to tooth surfaces and to the interstitial tooth spaces without deformation when the delivery system 10 is applied. The delivery system 10 can be applied without significant pressure.

The first layer 12 of the delivery system 10 may be comprised of a strip of material. Such first layer materials are described in more detail in U.S. Pat. Nos: 6,136,297; 6,096,328; 5,894,017; 5,891,453; and 5,879,691 and in U.S. Pat. Nos. 5,989,569 and 6,045,811; and in patent application US 2014/0178443 A1. The strip 12 serves as a protective barrier for the bleaching agent in the second layer 14. It prevents leaching and/or erosion of the second layer 14 by for example, the wearer's tongue, lips, and saliva. This allows the active agent in the second layer 14 to act upon the tooth surfaces 22 of the oral cavity for the intended period of time, from several minutes to several hours.

The following description of strip of material may apply to the delivery systems 10 with the strip layer 12 as shown exemplary in Figs. 1 to 4 or any form of strips. The strip material may comprise polymers, natural and synthetic woven materials, non-woven material, foil, paper, rubber and combinations thereof. The strip of material may be a single layer of material or a laminate of more than one layer. Regardless of the number of layers, the strip of material is substantially water insoluble. The strip material may also be water impermeable. Suitable strip material may be any type of polymer or combination of polymers that meet the required flexural rigidity and are compatible with oral care substances. Suitable polymers include, but are not limited to, polyethylene, ethylvinylacetate, polyesters, ethylvinyl alcohol and combinations thereof. Examples of polyesters include Mylar® and fluoroplastics such as Teflon®, both manufactured by Dupont. In particular, the material used as strip material is polyethylene. The strip of material may be generally less than about 1 mm (millimeter) thick, in particular less than about 0.05 mm thick, more particular from about 0.001 to about 0.03 mm thick. A polyethylene strip of material is generally less than about 0.1 mm thick and preferably from about 0.005 to about 0.02 mm thick.

In certain embodiments, the present invention may comprise a dissolvable film, which can be adhered to the oral cavity thereby releasing an active, the dissolvable film comprising water-soluble polymers, one or more polyalcohols, and one or more actives. In addition to one or more actives, a dissolvable film may contain a combination of certain plasticizers or surfactants, colorants, sweetening agents, flavors, flavor enhancers, or other excipients commonly used to modify the taste of formulations intended for application to the oral cavity. The resulting dissolvable film is characterized by an instant wettability which causes the dissolvable film to soften soon after application to the mucosal tissue, thus preventing the user from experiencing any prolonged adverse feeling in the mouth, and a tensile strength suitable for normal coating, cutting, slitting, and packaging operations.

The dissolvable film may comprise a water-soluble polymer or a combination of water-soluble polymers, one or more plasticizers or surfactants, one or more polyalcohols, and an active.

The polymers used for the dissolvable film include polymers which are hydrophilic and/or water-dispersible. Examples of polymers that can be used include polymers that are water-soluble cellulose-derivatives, such as hydroxypropylmethyl cellulose, hydroxyethyl cellulose, or hydroxypropyl cellulose, either alone, or mixtures thereof. Other optional polymers, without limiting the invention, include polyvinyl pyrrolidone, carboxymethyl cellulose, polyvinyl alcohol, sodium alginate, polyethylene glycol, natural gums like xanthane gum, tragacantha, guar gum, acacia gum, arabic gum, water-dispersible polyacrylates like polyacrylic acid, methylmethacrylate copolymer, carboxyvinyl copolymers. The concentration of the water-soluble polymer in the final film can very between 20 and 75% (w/w), or between 50 and 75% (w/w).

The surfactants that may be used for the dissolvable film may be one or more nonionic surfactants. When a combination of surfactants is used, the first component may be a polyoxyethylene sorbitan fatty acid ester or an ALPHA -hydro- OMEGA -hydroxypoly (oxyethylene)poly(oxypropylene)poly(oxyethylene) block copolymer, while the second component may be a polyoxyethylene alkyl ether or a polyoxyethylene castor oil derivative. In certain embodiments, the HLB value of the polyoxyethylene sorbitan fatty acid ester should be between 10 and 20, whereby a range of 13 to 17 may also be used. The ALPHA -hydro- OMEGA -hydroxypoly(oxyethylene)poly(oxypropylene) poly(oxyethylene) block copolymer may contain at least about 35 oxypropylene-units, and in certain embodiments not less than about 50 oxypropylene-units.

The polyoxyethylene alkyl ether may an HLB value between 10 and 20, and in certain embodiments an HLB value of not less than 15 may be used. The polyoxyethylene castor oil derivative may have an HLB value of 14-16.

In order to achieve the desired instant wettability, the ratio between the first and second component of the binary surfactant mixture may be kept within 1:10 and 1:1, or between 1:5 and 1:3.

The total concentration of surfactants in the dissolvable film depends on the properties of the other ingredients, but usually may be between 0.1 and 5% (w/w).

The polyalcohol can be used to achieve a desired level of softness of the dissolvable film. Examples of polyalcohols include glycerol, polyethylene glycol, propylene glycol, glycerol monoesters with fatty acids or other pharmaceutically used polyalcohols. The concentration of the polyalcohol in the dry film usually ranges between 0.1 and 5% (w/w).

The shape of the strip of material may be any shape and size that covers the desired oral surface. For example the strip may have rounded corners to avoid irritation of the soft tissue of the oral cavity. "Rounded corners," as used herein means not having any sharp angles or points for example one or more angles of 135° or less. The length of the strip of material maybe from about 2 cm (centimeter) to about 12 cm, in particular from about 4 cm to about 9 cm. The width of the strip of material will also depend on the oral surface area to be covered. The width of the strip is generally from about 0.5 cm to about 4 cm, in particular from about 1 cm to about 2 cm. The strip of material may be worn as a patch on one or several teeth to treat a localized condition.

The strip material may contain shallow pockets. When the composition is coated on a strip of material, bleach agents and/or oral care actives, fill shallow pockets to provide reservoirs of additional bleach agents and/or oral care actives. Additionally the shallow pockets help to provide texture to the delivery system. The strip of material may have an array of shallow pockets. Generally the shallow pockets are approximately 0.4 mm across and about 0.1 mm deep. When shallow pockets are included in the strip of material and compositions herein are applied to it in various thicknesses, the overall thickness of the delivery system is less than about 1 mm, in particular the overall thickness is less than about 0.5 mm.

Flexural stiffness is a material property that is a function of a combination of strip of material thickness, width and material modulus of elasticity. The test described below is a method for measuring the rigidity of for example polyolefin film and sheeting. It determines the resistance to flexure of a sample by using a strain gauge affixed to the end of a horizontal beam. The opposite end of the beam presses across a strip of the sample to force a portion of the strip into a vertical groove in a horizontal platform upon which the sample rests. A microammeter wired to the strain gauge is calibrated in terms of deflection force. The rigidity of the sample is read directly from the microammeter and expressed as grams per centimeter of the sample strip width. Strip of material which is suitable to be used as delivery carrier of the oral compositions as disclosed herein may show a flexural stiffness of less than about 5 grams/cm as measured on a Handle-O-Meter, model #211-300, available from Thwing-Albert Instrument Company of Philadelphia, PA as per test method ASTM D2923-95. The strip may have a flexural stiffness less than about 3 grams/cm, in particular less than about 2 grams/cm and more particular a flexural stiffness from about 0.1 to about 1 grams/cm. Generally, the flexural stiffness of the strip of material is substantially constant and does not change during normal use. For example, the strip of material does not need to be hydrated for the strip to achieve the low flexural stiffness in the above-specified ranges. This relatively low stiffness enables the strip of material to cover the contours of the oral surface with very little force being exerted. That is, conformity to the contours of the oral surface of the wearer's mouth is maintained because there is little residual force within the strip of material to cause it to return to its shape just prior to its application to the oral surface, i.e. substantially flat. The strip of material's flexibility enables it to contact soft tissue over an extended period of time without irritation; such that the strip of material does not require continuous pressure for retention against the oral surface.

The delivery systems as used herein may comprise an adhesion means such that they are capable of adhesion to oral surfaces especially the teeth. This adhesion means may be provided by the present oral compositions herein or the adhesion means may be provided independently of the oral compositions herein (for example the adhesion means is a separate phase from the oral compositions herein where the oral compositions may or may not also have an adhesive means). Preferably, the strip of material may be held in place on the oral surface by adhesive attachment provided by the present oral composition. Preferably, the viscosity and general tackiness of the multi-phase composition to dry surfaces cause the strip to be adhesively attached to the oral surface without substantial slippage from the frictional forces created by the lips, teeth, tongue, and other oral surfaces rubbing against the strip of material while talking drinking, etc. However, this adhesion to the oral surface is low enough to allow the strip of material to be easily removed by the wearer by simply peeling off the strip of material using one's finger. The delivery system may be easily removable from the oral surfaces without the use of an instrument, a chemical solvent or agent or excess friction.

In addition or alternatively, the strip of material may be held in place on the oral surface by adhesive means and attachment provided by the delivery carrier itself. For example, the strip of material can extend, attach, and adhere to the oral soft tissue. In addition or alternatively, an adhesive can be applied to that portion of the strip of material that will attach the delivery systems to the oral soft tissue. The delivery carrier may also be attached to the oral cavity by physical interference or mechanical inter-locking between the delivery carrier and the oral surfaces including the teeth. In addition or alternatively the strip of material may be held in place by an adhesion mans that is independent of the oral composition of the present inventions herein, as disclosed in WO 03/015656.

Suitable adhesion means are known to the skilled person. When the adhesive means, -if present- is provided by an adhesive, the adhesive may be any adhesive which may be used to adhere materials to the tooth surface or to a surface of the oral cavity surfaces. Suitable adhesives include, but are not limited to, skin, gum and muco adhesives, and should be able to withstand the moisture, chemicals and enzymes of the oral environment for long enough for the oral care actives and/or bleach to take effect, but may be soluble and/or biodegradable thereafter. Suitable adhesives may for example comprise water soluble polymers, hydrophobic and/or non-water soluble polymers, pressure and moisture sensitive adhesives, e.g. dry adhesives which become tacky upon contact with the mouth environment, e.g. under the influence of moisture, chemicals or enzymes etc. in the mouth. Suitable adhesives include natural gums, synthetic resins, natural or synthetic rubbers, those gums and polymers listed above under "Thickening Agents", and various other tacky substances of the kind used in known adhesive tapes, those known from US-A-2,835,628.

The delivery carrier, such as a strip as shown for example in Figs. 1 to 4, may be formed by several of the film making processes known in the art. For example a strip of polyethylene is made by a blown process or a cast process. Other processes including extrusion or processes that do not affect the flexural rigidity of the strip of material are also feasible. In addition, the present oral compositions forming a second layer onto the strip may be incorporated onto the strip during the processing of the strip and/or the present oral composition may be a laminate layer on the strip. The second layer attached to the strip of such a delivery system as disclosed above comprises a safe and effective amount of the present oral composition described herein.

In addition, the delivery system may comprise an optional release liner. Such a release liner may be formed from any material which exhibits less affinity for the second layer oral composition than the second layer oral composition exhibits for itself and for the first layer strip of material. The release liner may comprise a rigid sheet of material such as polyethylene, paper, polyester, or other material, which is then coated with a nonstick type material. The release liner may be cut to substantially the same size and shape as the strip of material or the release liner may be cut larger than the strip of material to provide a readily accessible means for separating the material from the strip. The release liner may be formed from a brittle material that cracks when the strip is flexed or from multiple pieces of material or a scored piece of material. Alternatively, the release liner may be in two overlapping pieces such as a typical adhesive bandage design. A description of materials suitable as release agents is found in Kirk-Othmer, Encyclopedia of Chemical Technology, Fourth Edition, Volume 21, pp. 207-218, incorporated herein by reference.

In certain embodiments the delivery carrier may be a deformable strip of material having a yield point and thickness such that the strip of material such that it at least partially conforms to a shape of a tooth under a pressure less than about 250,000 Pascals as it has been found that wearers will press a strip onto each tooth using one fingertip having about one square centimeter surface area. They typically apply force at each tooth for one second or less with a typical application pressure ranging from about 100,000 Pascals to about 250,000 Pascals.

A preferred strip of material has visco-elastic properties which enable it to creep as well as bend in order to conform across several teeth and around the arch of the wearer's mouth. It is important that the necessary shaping occurs under minimum normal force being applied by the wearer.

The oral composition may also be applied to the tooth surface and may be covered with the deformable strip before or after it has been shaped. In addition or alternatively, the oral composition may be applied to the deformable strip as pre-coating and may be applied together with the strip to the tooth surface before or after the deformable strip has been shaped, wherein the strip is applied such that when the delivery system is placed on a surface of the tooth, the oral composition contacts the tooth surface providing an active onto the tooth surface. In addition or alternatively, the deformable strip of material may be applied to the teeth with a force sufficient to shape the delivery carrier such that it at least partially conforms to the shape of the teeth, then the shaped strip of material may be removed from the tooth surface, the oral care composition may be applied to the shaped strip of material, and the shaped strip of material may be re-applied to the tooth surface such that it at least partially conforms to a shape of the tooth and contacts the oral care composition against the tooth surface. If the deformable strip is applied together with the oral composition to the tooth surface the oral composition may also comprise adhesive agents to hold the delivery system in place for a sufficient time to allow the active of the oral composition to act upon the surface. The oral composition, if used together with a deformable strip, may have an extrusion resistance sufficient to withstand a normal force applied to shape the deformable strip of material so that the substance is not substantially extruded from between the deformable strip of material and the surface during manual shaping of the deformable strip of material. By "substantially extruded from" is meant that at least 50% or more of the oral composition is extruded from between the deformable strip of material and the tooth and adjoining soft tissue surfaces.

The deformable strip of material may be preferably made of a permanently deformable material, such as wax, putty, tin or foil, as a single layer or a combination of layers or materials, such as a laminate. Preferably, the deformable strip may be wax, such as #165 sheet wax formulated and manufactured by Freeman Mfg. & Supply Co. of Cleveland, Ohio. This particular wax readily conforms to the shape of a tooth under a pressure of about 133,000 Pascal which is the pressure generated when the wearer applies a normal force of about 3 pounds (1.36 kg) over an area of about one square centimeter. The preferred deformable strip may have a nominal film thickness of about 0.8 mm, wherein the deformable strip may be substantially flat and rectangular in shape with rounded corners. The deformable strip of material may have a length sufficient to cover a plurality of adjacent teeth while conforming to the curvature of the wearer's mouth and gaps between the adjacent teeth. If the deformable strip of material includes the oral composition coated thereon, the oral composition may have an overall thickness less than about 1.5 mm. Deformable strips as disclosed herein may also be used as the material for the strip of material 12 shown in Figs. 1 to 4. Thus, general features of strip of material as described above for example with respect to Figs. 1 to 4 may also apply to the deformable strip of material. In addition, a release liner and/or shallow pockets may also be combined with a deformable strip of material.

Alternatively, the present oral compositions may be used in combination with a delivery carrier including a dental tray and/or foam material. Dental trays are well known in the whitening art and an example dental tray 30 is shown in Fig. 5. The general process for preparing dental trays 30 is known in the art. Dentists have traditionally utilized several different types of dental appliances for bleaching teeth.

The first type is a rigid appliance which is fitted precisely to the patient's dental arches. For example, an alginate impression which registers all teeth surfaces plus gingival margin is made and a cast is promptly made of the impression. If reservoirs are desired they are prepared by building a layer of rigid material on the cast on specific teeth surfaces to be treated. A dental tray is then vacuum formed from the modified cast using conventional techniques. Once formed, the tray is preferably trimmed barely shy of the gingival margin on both buccal and lingual surfaces. Enough tray material should be left to assure that all of the tooth will be covered to within about ¼ to about ⅓ mm of the gingival border upon finishing and beveling the tray periphery. One can scallop up and around interdental papilla so that the finished tray does not cover them. All tray edges are preferably smoothed so that the lip and tongue will not feel an edge prominence. The resulting tray, provides a perfect fit of the patient's teeth optionally with reservoirs or spaces located where the rigid material was placed on the stone cast. Dental trays may comprise of soft transparent vinyl material having a preformed thickness from about 0.1 cm to about 0.15 cm. Soft material is more comfortable for the patient to wear. Harder material (or thicker plastic) may also be used to construct the tray.

A second type of rigid custom dental appliance is an "oversized" rigid custom dental appliance. The fabrication of rigid, custom dental appliances entails fabricating cast models of the patient's dental arch impressions, and heating and vacuum-forming a thermoplastic sheet to correspond to the cast models of a patient's dental arches. Thermoplastic films are sold in rigid or semi rigid sheets, and are available in various sizes and thickness. The dental laboratory fabrication technique for the oversized rigid dental appliance involves augmenting the facial surfaces of the teeth on the cast models with materials such as die spacer or light cured acrylics. Next, thermoplastic sheeting is heated and subsequently vacuum formed around the augmented cast models of the dental arch. The net effect of this method results in an "oversized" rigid custom dental appliance.

A third type of rigid custom dental appliance, used with less frequency, is a rigid bilaminated custom dental appliance fabricated from laminations of materials, ranging from soft porous foams to rigid, non-porous films. The nonporous, rigid thermoplastic shells of these bilaminated dental appliances encase and support an internal layer of soft porous foam.

A fourth type of dental tray replaces rigid custom dental appliances with disposable U-shaped soft foam trays, which may be individually packaged, and which may be saturated with a pre-measured quantity of the oral composition of the present invention. The soft foam material is generally an open celled plastic material. Such a device is commercially available from Cadco Dental Products in Oxnard, Calif under the tradename VitalWhite™. These soft foam trays may comprise a backing material (e.g. a closed cell plastic backing material) to minimize the elution of the bleaching agent from the device, into the oral cavity to minimize ingestion by the patient and/or irritation of the oral cavity tissues. Alternatively, the soft foam tray is encased by a nonporous flexible polymer or the open cell foam is attached to the frontal inner wall of the dental appliance and/or the open cell foam is attached to the rear inner wall of the dental appliance. Those of ordinary skill in the art will readily recognize and appreciate, that the present oral compositions must be thick enough not to simply run out between the open cell structure of the foam and must be thin enough to slowly pass through the open cell foam over time. In other words, the open cell foam material has an internal structural spacing sized relative to the viscosity of the oral compositions to absorb and allow the oral composition to pass there through.

An example of a closed cell material is a closed-celled polyolefin foam sold by the Voltek division of Sekisui America Corporation of Lawrence, Mass. under the tradename Volora which is from 1/32" to 1/8" in thickness. A closed cell material may also comprise of a flexible polymeric material. An example of an opened cell material is an open celled polyethylene foam sold by the Sentinel Foam Products division of Packaging Industries Group, Inc. of Hyannis, Mass. under the tradename Opcell which is from 1/16" to 3/8" in thickness. Other open cell foam useful herein include hydrophilic open foam materials such as hydrogel polymers (e.g Medicell™ foam available from Hydromer, Inc. Branchburg, J.J.). Open cell foam may also be hydrophilic open foam material imbibed with agents to impart high absorption of fluids, such as polyurethane or polyvinylpyrrolidone chemically imbibed with various agents.

Preparation of the present Oral Compositions in form of an emulsion

Principally, preparation of emulsions is well known in the art and any suitable manufacturing process can be used to make the emulsion; see for example, Remmingtion: the Science and Practice of Pharmacy, 19th ed., Vol. II, Chapters 20, 80, 86, etc.. Generally, the components are separated into those that are oil-soluble and those that are water-soluble. These are dissolved in their respective solvents by heating if necessary. The two phases are then mixed and the product is stirred and cooled. After combining the phases, the present multiphase compositions preferably in the form of water-in-oil emulsions may be agitated or sheared by various methods, including shaking, intermittent shaking, high shear mixing, or by using high speed mixers, blenders, colloid mills, homogenizers, or ultrasonic techniques. Various test methods are available to confirm the type of multiphase compositions preferably in the form of a water-in-oil emulsion that were prepared. These test methods include the dilution test, conductivity test, microscopy, and the dye-solubility test methods. Further description of test methods are disclosed in Remington: The Science and Practice of Pharmacy, 19th ed., volume 1, 1995, pp. 282-283.

In particular, the multiphase compositions preferably in the form of a water-in-oil emulsion of the oral composition as disclosed herein is suitably made as follows: Dissolve the bleach active in the aqueous phase; then combine the aqueous phase and the hydrophobic phase in a mixing vessel and mix well with any means known within the art, for example, a Speed-mixer (from Flactek Inc., Landrum, SC) may be used to make the multiphase compositions preferably in the form of a water-in-oil emulsion of the present invention. The mixing procedure of the SpeedMixer™ series is based on the double rotation of the mixing cup using a dual asymmetric centrifugal mixing. This combination of centrifugal forces acting on different levels enables very rapid mixing of the entire cup. Optionally the oral composition may be heated, if necessary to facilitate solving of the bleaching active or the mixing. Flavorants or sweeteners may also be added to one of the phases of the oral composition, as desired. Thereafter the oral composition may be added to the delivery carrier, as desired.

Methods of Using the Oral Compositions and/or Delivery Systems

The present invention can be applied to the teeth of a consumer in the dental office by a dental professional, or can be used at home by the consumer. Generally, the recommended treatment period is, a sufficient period of time to achieve whitening.

In practicing the present invention, the user applies the oral composition herein that contains the bleaching agent to obtain the desired effect, e.g., whitening, to one or more teeth. The oral composition can be applied with a paint-on device, a syringe or unit dose syringe, squeezable tube, a brush, a pen or brush tip applicator, a doe's foot applicator, or the like, or even with the fingers. The oral composition be applied directly or can also be combined with a delivery carrier, e.g. such as a strip of material, a dental tray, and/or a sponge material, and thereafter applied to the teeth. Preferably, the oral compositions and/or delivery systems herein are almost unnoticeable when applied to the teeth. After a desired period of time has elapsed, any residual oral composition may be easily removed by wiping, brushing or rinsing the oral surface.

In general, it is not necessary to prepare the teeth before applying the present oral composition. For example, the user may choose to brush the teeth or rinse the mouth before applying the oral compositions of the present invention, but the surfaces of the oral cavity are neither required to be clean, nor to be dried nor to be excessively wet with saliva or water before the application. However, it is believed that adhesion to the tooth enamel surfaces will be improved if the teeth are dry prior to application.

Dental tray appliances may be used as follows. The patient or dental professional dispenses the present oral composition into a soft or rigid dental appliance and then the participant places the appliance over the participant's dental arch (or fits the device around his or her teeth to keep the tray in position). Generally, the recommended treatment period is a sufficient period of time to achieve whitening as disclosed above. At the end of the treatment period, the dental appliance is removed, cleaned with water to remove any remaining oral composition, and then stored until the next application.

The above-described oral compositions and delivery systems may be combined in a kit which comprises: 1. present oral composition and 2. instructions for use; or which comprises: 1. present oral composition, 2. instructions for use, and 3. a delivery carrier. In addition, if the tooth shall be radiated by electromagnetic radiation, the kit may further comprise an electromagnetic radiation source of the appropriate wavelength and instruction for use, so that the kit can be used by consumers in a convenient manner.

The oral compositions of this invention are useful for both human and other animals (e.g. pets, zoo, or domestic animals) applications.

Electromagnetic Radiation Treatment

The oral compositions as disclosed herein may be used to whiten teeth and/or removing stain from tooth surfaces. In addition, the bleaching efficacy may be further increased by directing electromagnetic radiation of a suitable wavelength toward at least one tooth. A suitable wavelength may be every wavelength which corresponds to a maximum absorption band of the tooth and/or the tooth stain to be bleached. For example the oral composition may be radiated with electromagnetic radiation with one or more wavelengths in the range of from about 200 nm to about 1700 nm. The electromagnetic radiation may be directed toward at least one tooth. In addition more than one tooth may be irradiated in parallel. The electromagnetic radiation may have a peak intensity at a wavelength in the range of from about 400, 405, 410, 415, 420, 425, 430, 435, 440, or 445, 446 nm to about 450, 455, 460, 465, 470, 475, 480, 481, 485, 490, 495, or 500 nm or any other numerical range which is narrower and which falls within such broader numerical range, as if such narrower numerical ranges were all expressly written herein. Preferably the electromagnetic radiation has a peak intensity at a wavelength in the range of from about 425 nm to about 475 nm, and more preferred from about 445 nm to about 465 nm, or wherein the peak intensity wavelength of the electromagnetic radiation is similar to the wavelength at which the stain absorbs the most electromagnetic radiation. Electromagnetic radiation may be directed toward at least one tooth for partial or whole wearing time of the oral composition or after the oral composition has been removed from the tooth. Electromagnetic radiation may be applied at least for a sufficient period of time for whitening, e.g. for at least about 1 minute, preferably for at least about 5 minutes, and more preferably for at least about 10 min. The electromagnetic radiation may be applied using the procedure disclosed in US 2013/0295525. Preferably the oral composition as disclosed herein is applied to at least one tooth and maintained on the at least one tooth for a first period of time; after the first period of time an electromagnetic radiation is directed toward the at least one tooth for a second period of time, wherein the first period of time has a duration greater than 50%, preferably 80% of a total duration of the first and second periods of time; and finally, the oral composition is removed from the at least one tooth.

Suitable sources of electromagnetic radiation include the source described herein in the section titled "Clinical Protocol".

If the oral compositions as disclosed are combined with electromagnetic radiation, the oral compositions are preferably transparent or translucent to electromagnetic radiation with wavelengths from about 200 nm to about 1700 nm, preferably transparent or translucent to electromagnetic radiation wavelengths as disclosed above. Preferably, the oral compositions as disclosed herein allow from about 10%, 20%, or 30 % to about 40%, 50%, 60%, 70%, 80%, 90%, or 100% of electromagnetic radiation from about 400 nm to about 500 nm to pass through as measured by Spectrophotometry. Preferably, the oral compositions as disclosed herein when applied in a thickness of from about 0.0001cm, 0.001cm, or 0.01cm to about 0.01cm, 0.1cm, or 0.5cm thickness allow from about 10%, 20%, or 30 % to about 40%, 50%, 60%, 70%, 80%, 90%, or 100% of electromagnetic radiation from about 400 nm to about 500 nm to pass through. In addition or alternatively, the oral compositions as disclosed herein when preferably applied in an amount from about 0.0001g, 0.001g, or 0.01g to about 0.01g, 0.1g, 1g, or 5g, on a delivery carrier or tray with a surface area from about 1cm$^2$ to about 20cm$^2$, allow from about 10%, 20%, or 30% to about 40%, 50%, 60%, 70%, 80%, 90%, or 100% of electromagnetic radiation from about 400 nm to about 500 nm to pass through.

The electromagnetic radiation impinging on the surface of the tooth or outer surface of the carrier, preferably a strip, in the wavelength range from about 200 nm to about 1700nm, or from about 200 nm to about 1100 nm, or from about 400 to about 500 nm may range in intensity from about 0.1, 1, 5, 10, 25, 50, 75, or 100 mW/cm2 to about 500, 250, 225, 205, 200, 175, 150, 125, 100, 75, 50, 25, 10, or 5, 1, 0.1 mW/cm2 or any other numerical range which is narrower and which falls within such broader numerical range, as if such narrower numerical ranges were all expressly written herein.

Procedure to measure intensity of electromagnetic radiation

The intensity of the electromagnetic radiation can be measured using a spectrometer (USB 2000+ from Ocean Optics) connected to a UV-VIS 200 micron fiber-optic cable with a cosine corrector at the tip (OP 200-2-UV-VIS from Ocean Optics). The spectrometer is connected to a computer running the spectrometer software (Oceanview 1.3.4 from Ocean Optics). The tip of the fiber-optic cable is held pointing toward the light source at the location where the light intensity is to be measured. The photons collected at the detector surface are guided via the fiber-optic cable to the charge-coupled device in the spectrometer (CCD). The CCD counts photons arriving to the CCD during a pre-determined time period at each wavelength from 200 nm to 1100 nm, and uses a software algorithm to convert these photon counts to spectral irradiance (mW/cm$^2$/nm). The spectral irradiance is integrated from 200 nm to 1100 nm by the software to yield the Absolute Irradiance (mW/cm$^2$), which is the intensity of electromagnetic radiation from 200 nm to 1100 nm. The spectral irradiance is integrated from 400 nm to 500 nm by the software to yield the Absolute Irradiance (mW/cm$^2$), which is the intensity of electromagnetic radiation from 400 nm to 500 nm.

Preferably, the amount of oral composition applied to the tooth or the thickness of the oral composition applied to the tooth does not decrease the electromagnetic radiation transmission from about 400 nm to about 500 nm by more than about 10%.

For consumer convenience the oral composition as disclosed herein are provided as a Kit comprising the bleaching oral composition as disclosed herein, a delivery carrier for easier application, an electromagnetic radiation source emitting electromagnetic radiation in a suitable wavelength, and instructions for use.

EXAMPLES

The following non-limiting examples further describe embodiments. Many variations of these examples are possible without departing from the scope of the invention.

These oral compositions were made as described below.

Specifically, 500 gram batches of Example-I-A, Example-II-A, B, and C, Comparative Example-I, and Example-III A, B, C, D were made by weighing the aqueous solution of hydrogen peroxide (H2O2) and petrolatum into a Speedmixer container, and mixing in a Speedmixer at 800RPM for 5 seconds, 1200 RPM for 5 seconds, and 1950 RPM for 2 minutes. The walls of the container were then scraped down with a plastic spatula, and the contents were mixed a second time at 800RPM for 5 seconds, 1200 RPM for 5 seconds, and 1950 RPM for 2 minutes. The walls of the container were then scraped down with a plastic spatula, and the contents were mixed a third time at 800RPM for 5 seconds, 1200 RPM for 5 seconds, and 1950 RPM for 2 minutes.

Also, a 500 gram batch of Example-III E was made by first weighing the polyethylene and mineral oil into a Speedmixer container, heating it in an oven set at 95°C for about 3 hours, mixing with a spatula for about 30 seconds, followed by mixing in a Speedmixer at 800RPM for 5 seconds, 1200 RPM for 5 seconds, and 1950 RPM for 2 minutes, and cooling overnight at room temperature. Next, the aqueous solution of H2O2 was added and mixed in a Speedmixer at 800RPM for 5 seconds, 1200 RPM for 5 seconds, and 1950 RPM for 2 minutes. The walls of the container were then scraped down with a plastic spatula, and the contents were mixed a second time at 800RPM for 5 seconds, 1200 RPM for 5 seconds, and 1950 RPM for 2 minutes. The walls of the container were then scraped down with a plastic spatula, and the contents were mixed a third time at 800RPM for 5 seconds, 1200 RPM for 5 seconds, and 1950 RPM for 2 minutes.

Also, a 500 gram batch of Example-III F was made by first weighing the microcrystalline wax and mineral oil into a Speedmixer container, heating it in an oven set at 95°C for about 3 hours, mixing with a spatula for about 30 seconds, followed by mixing in a Speedmixer at 800RPM for 30 seconds, and cooling overnight at room temperature. Next, the aqueous solution of H2O2 was added and mixed in a Speedmixer at 800RPM for 5 seconds, 1200 RPM for 5 seconds, and 1950 RPM for 2 minutes. The walls of the container were then scraped down with a plastic spatula, and the contents were mixed a second time at 800RPM for 5 seconds, 1200 RPM for 5 seconds, and 1950 RPM for 2 minutes. The walls of the container were then scraped down with a plastic spatula, and the contents were mixed a third time at 800RPM for 5 seconds, 1200 RPM for 5 seconds, and 1950 RPM for 2 minutes.

Example I

Oral composition of Example I-A was made using the procedure described above and formulated with a 35% aqueous solution of hydrogen peroxide.

| Example I | A (Wt %) |
|---|---|
| 35% aqueous solution $H_2O_2$[1] | 0.2857 |
| Petrolatum[2] | 99.7143 |
| total | 100.00 |
| % $H_2O_2$ in total oral composition | 0.099995 |
| RATIO* | 350.02 |
| *RATIO of the concentration in weight percent of $H_2O_2$ present in the aqueous phase to the net concentration in weight percent of $H_2O_2$ present in the overall composition [1]ultra Cosmetic Grade from Solvay, Houston, Texas [2]G-2191 Grade from Sonneborn, LLC., Parsippany, NJ | |

Example II

Oral compositions of Example II were made using the procedure described above and formulated with 1) a 17.5% aqueous solution of hydrogen peroxide, and 2) a 5% solution of hydrogen peroxide:

| Example II | A (Wt %) | B (Wt%) | C (Wt%) |
|---|---|---|---|
| 17.5% aqueous sol. $H_2O_2$[3] | 0.5714 | 0.0000 | 0.0000 |
| 5% aqueous sol. $H_2O_2$[3] | 0.0000 | 1.0000 | 2.0000 |
| Petrolatum[4] | 99.4286 | 99.0000 | 98.0000 |
| total | 100.0 | 100.0 | 100.0 |
| % $H_2O_2$ in total composition | 0.099995 | 0.0500 | 0.1000 |
| RATIO* | 175.01 | 100.00 | 50.00 |
| *RATIO of the concentration in weight percent of $H_2O_2$ present in the aqueous phase to the net concentration in weight percent of $H_2O_2$ present in the overall composition<br>[3]ultra Cosmetic Grade from Solvay (Houston, Texas) diluted with water<br>[4]G-2191 Grade from Sonneborn, LLC., Parsippany, NJ | | | |

Example III

Oral compositions of Example III were made using the procedure described above and formulated with 1) a 35% aqueous solution of hydrogen peroxide of different chemical grades as well as 2) different materials used as the hydrophobic phase.

| Example III | A (Wt %) | B (Wt %) | C (Wt %) | D (Wt %) | E (Wt %) | F (Wt %) |
|---|---|---|---|---|---|---|
| 35% aqueous sol. $H_2O_2$[5] | 0.2857 | | | 0.2857 | 0.2857 | 0.2857 |
| 35% aqueous sol. $H_2O_2$[6] | | 0.2857 | | | | |
| 35% aqueous sol. $H_2O_2$ [7] | | | 0.2857 | | | |
| Petrolatum[8] | | 99.7143 | 99.7143 | | | |
| Petrolatum[9] | 99.7143 | | | | | |
| Petrolatum[10] | | | | 99.7143 | | |
| Mineral oil[11] | | | | | 79.7143 | 49.7143 |
| Polyethylene[12] | | | | | 20.00 | |
| Microcrystalline Wax[13] | | | | | | 50.00 |
| total | 100.0 | 100.0 | 100.0 | 100.0 | 100.0 | 100.0 |
| % $H_2O_2$ in total compos. | 0.099995 | 0.099995 | 0.099995 | 0.099995 | 0.099995 | 0.099995 |
| RATIO* | 350.02 | 350.02 | 350.02 | 350.02 | 350.02 | 350.02 |
| *RATIO of the concentration in weight percent of $H_2O_2$ present in the aqueous phase to the net concentration in weight percent of $H_2O_2$ present in the overall composition<br>[5]ultra Cosmetic Grade from Solvay, Houston, Texas<br>[6]Technical Grade from Solvay, Houston, Texas<br>[7]Technical grade from Solvay Stabilized with added Stabilizers<br>[8]G-2191 Grade from Sonneborn, LLC., Parsippany, NJ<br>[9]G-1958 Grade from Sonneborn, LLC., Parsippany, NJ<br>[10]G-2218 Grade from Sonneborn, LLC., Parsippany, NJ<br>[11]Kaydol grade from Sonneborn, LLC., Parsippany, NJ<br>[12]400 Grade from Baker-Hughes, Houston, TX, dissolved into the mineral oil at about 95C<br>[13]W835 Grade from Sonneborn, LLC., Parsippany, NJ, dissolved into the mineral oil at about 95°C. | | | | | | |

COMPARATIVE EXAMPLES

Comparative Example I

Comparative Example I was made using the procedure described above and formulated with no bleaching agent.

| Comparative Example I | (Wt %) |
|---|---|
| Petrolatum[14] | 100.0000 |
| total | 100.0000 |
| % $H_2O_2$ in total oral composition | 0.0000 |
| [14]G-2191 Grade from Sonneborn, LLC., Parsippany, NJ | |

Bleaching Efficacy of Example-IA versus Comparative Example-I

The bleaching efficacy of Example-IA and Comparative Example-I were measured per the clinical protocol disclosed herein. Specifically, this was a randomized, single-center, two-treatment, parallel group, clinical study conducted on 39 adults who had never had a professional, over-the-counter or investigational tooth bleaching treatment. All subjects were at least 18 years old, had all four measurable maxillary incisors, and had no self-reported tooth sensitivity. Subjects were randomized to study treatments based on L* and b* color values and age. Participants were assigned to one of two treatment groups:

• Example-IA (22 subjects, mean L* of 74.1 and mean b* of 15.6) or

• Comparative Example-I (17 subjects, mean L* of 74.2 and mean b* of 15.2)

The maxillary anterior teeth of the subjects were treated with the oral composition they were assigned for 60 minutes once daily using a strip of polyethylene as a delivery carrier. The polyethylene strips were 66mm x 15mm in size and 0.0178mm thick. 0.6 grams to 0.8 grams of the oral compositions were applied across each strip of polyethylene prior to applying to the maxillary anterior teeth.

Distribution of the assigned maxillary strips and all applications were supervised by a clinical site staff. For each treatment, subjects wore a strip with the oral composition they were assigned for a total of 60 minutes. After 50 minutes of each strip wear, a trained hygienist applied electromagnetic radiation toward the facial surfaces of the maxillary anterior teeth for 10 minutes. The electromagnetic radiation was directed toward the teeth through the strip and through the oral composition. The electromagnetic radiation was delivered using the source of electromagnetic radiation described herein in the section titled "Clinical Protocol". The intensity of the electromagnetic radiation from 400 nm to 500 nm measured at the central axis of each cone of electromagnetic radiation exiting at the exit surface of the transparent window through which the electromagnetic radiation passes toward the maxillary anterior teeth was measured to be about 205 mW/cm2 as measured by the method disclosed herein.

Digital images were collected at Baseline, and the day after the 3rd, 7th, 10th, and 14th treatments.

The group using Example-IA demonstrated a statistically significant (p<0.0001), incremental reduction in yellowness (-$\Delta$b*) at all tested time-points relative to Baseline; in addition, increase in lightness ($\Delta$L*) was observed in this group the day after seven, ten, and fourteen treatments (p<0.001).

The group using comparative Example-I did not differ from Baseline values after three, seven, and ten applications, and showed a small statistically significant (p=0.0007) decrease in yellowness (-$\Delta$b*) after fourteen treatments; no changes in lightness ($\Delta$L*) were detected.

Furthermore, the group on Example-IA demonstrated a larger decrease in yellowness -$\Delta$b*) compared to the group on comparative Example-I at all tested time-points.

| Table I shows the results in detail: Mean change in yellowness from baseline ($\Delta$b*) | Example-IA (0.099995% $H_2O_2$ delivered on a strip and used with an electromagnetic radiation source described herein in the section titled "Clinical Protocol") | Comparative Example-I (0% $H_2O_2$ delivered on same strip and used with same electromagnetic radiation source described herein in the section titled "Clinical Protocol") | % Improvement delivered by Example-IA over Comparative Example-I under same conditions |
|---|---|---|---|
| After 3 treatments (Day 4) | -0.607 | 0.073 | > 800% |
| After 7 treatments (Dav 8) | -1.45 | 0.005 | > 800% |
| After 10 treatments (Day 11) | -1.70 | -0.191 | > 800% |
| After 14 treatments (Dav 15) | -1.95 | -0.408 | > 400% |

These results clearly demonstrate the surprisingly high efficacy of Example-IA (delivered on a strip and used with electromagnetic radiation as disclosed herein) even though it has less than 0.1% $H_2O_2$.

The ratio of bleaching efficacy of Example-IA (delivered on a strip and used with electromagnetic radiation as disclosed herein) as measured per the clinical protocol as disclosed herein and calculated as -$\Delta$b* to the net weight percent of bleaching agent present in the overall oral composition was 6.07, 14.5, 17.0, and 19.5 after 3, 7, 10, and 14 treatments respectively.

These results also clearly demonstrate the surprisingly high efficacy of Example-IA (delivered on a strip and used with electromagnetic radiation as disclosed herein) relative to the comparative Example-I (delivered on same strip and used with the same electromagnetic radiation source).

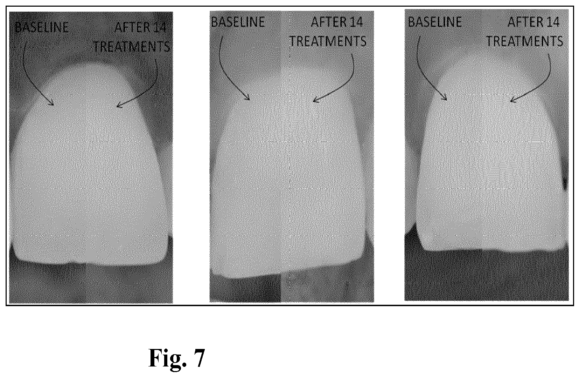

Fig. 7 shows images of example teeth treated with the bleaching oral composition of Example IA. RGB images were converted to black-and-white images. Images were taken before and after 14 treatments with oral composition of Example IA. Three teeth were shown, wherein the left side of the tooth shows its baseline visual appearance and the right side of the tooth shows its visual appearance after 14 treatments. It can be clearly seen that the treatment with Example IA oral composition visibly whitens the tooth surface. All three teeth appear whiter on the right side compared to the left side.

It is also surprising that none of the study subjects reported tooth-sensitivity despite experiencing the high efficacy of Example-IA (delivered on a strip and used with an electromagnetic radiation source as disclosed herein).

Comparative Example II

Comparative example II is a commercially available Crest Whitestrips tooth whitening strip product with 5.25% H2O2 (from Procter & Gamble, Cincinnati, OH, USA). This is an aqueous gel containing 5.25% hydrogen peroxide ($H_2O_2$); and since it is an aqueous gel, the ratio of the concentration in weight percent of H2O2 present in the aqueous phase to the net concentration in weight percent of H2O2 present in the overall composition is 1.

Bleaching Efficacy of Comparative Example II (Aqueous Gel with 5.25% $H_2O_2$)

The bleaching efficacy of a second comparative composition (Comparative Example II) containing a final concentration of 5.25% $H_2O_2$ in an aqueous gel was also measured per the clinical protocol disclosed herein except that the commercially available product specified in Comparative Example II was used. Specifically, the study for Comparative Example II was a controlled, single-center clinical trial. The target population was adult subjects with no previous history of tooth whitening. Participants were treated with the above comparative aqueous gel with 5.25% $H_2O_2$ (Comparative Example II) delivered on a strip of polyethylene. The group (20 subjects, mean L* of 72.8 and mean b* of 16.4) wore the strip for 60 minutes once daily for 14 days.

Digital images were obtained at Baseline, and the day after the 7th and 14th treatments.. The results of the group who wore the comparative Example II (aqueous gel with 5.25% $H_2O_2$) delivered on a strip for 60 minutes (same length of time as Example-IA in the clinical described previously) are shown in the table below.

Table II shows the results in detail:

| Mean change in yellowness from baseline ($\Delta b^*$) | Example-IA (composition of invention containing about 0.1% H2O2) (delivered on a strip for 60 minutes, and used with an electromagnetic radiation source described herein in the section titled "Clinical Protocol") | Comparative Example II (aqueous gel containing about 5.25% H2O2) (delivered on a strip for 60 minutes) |
|---|---|---|
| After 7 treatments (Day 8) | -1.45 | -0.985 |
| After 14 treatments (Day 15) | -1.95 | -1.43 |

After 7 treatments, the comparative Example II (aqueous gel with 5.25% $H_2O_2$, delivered on a strip for 60 minutes) produced a mean change in yellowness of -0.985 while Example-IA (also delivered on a strip, and used with an electromagnetic radiation source) delivered a mean change in yellowness of -1.45 even though it had approximately 5250% lower concentration of $H_2O_2$ vs. the aqueous gel (0.1% $H_2O_2$ Vs. 5.25% $H_2O_2$) used in Comparative Example II. Similarly after 14 treatments, the comparative Example II produced a mean change in yellowness of -1.43 while Example-IA delivered a mean change in yellowness of -1.95 even though it had approximately 5250% lower concentration of $H_2O_2$ Vs. the aqueous gel (0.1% $H_2O_2$ vs. 5.25% $H_2O_2$). It is worth noting that Comparative Example I which had the same electromagnetic radiation source disclosed herein but with 0.0% $H_2O_2$ delivered a mean change in yellowness of only 0.005 and -0.408 after 7 and 14 treatments respectively. These results also clearly demonstrate the surprisingly high efficacy of Example-IA (delivered on a strip and used with an electromagnetic radiation source as disclosed herein) even though it has approximately 5250% lower concentration of $H_2O_2$ Vs. the comparative aqueous gel (0.1% $H_2O_2$ Vs. 5.25% $H_2O_2$) used in Comparative Example II.

Also it is worth noting that the ratio of the concentration in weight percent of bleaching agent present in the aqueous phase to the net concentration in weight percent of bleaching agent present in the overall composition of the comparative example II is 1, while example IA has a ratio of 350.02.

The ratio of bleaching efficacy of Comparative Example II as measured per the clinical protocol as disclosed herein and calculated as -$\Delta b^*$ to the net weight percent of bleaching agent present in the overall oral composition was 0.19 and 0.27, after 7 and 14 treatments respectively. This is lower than the ratio of bleaching efficacy of Example-IA (delivered on a strip and used with an electromagnetic radiation source as disclosed herein) as measured per the clinical protocol as disclosed herein and calculated as -$\Delta b^*$ to the net weight percent of bleaching agent present in the overall oral composition which was measured to be 14.5 and 19.5, after 7 and 14 treatments respectively.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

**Claims**

1. A kit for whitening teeth comprising:

   I) a delivery carrier,
   II) an oral composition in the form of a water-in oil emulsion for whitening teeth, comprising

       i) from 0.01% to 1% by weight of an aqueous phase;
       ii) at least 99% by weight of a continuous, hydrophobic phase;
       iii) at least one oral care active agent comprised in the aqueous phase; wherein the active agent is a bleaching agent and the net concentration of the bleaching agent is up to 0.1% by weight of the oral composition; and

   wherein the oral composition is applicable to the delivery carrier; and
   III) an electromagnetic radiation source capable of directing electromagnetic radiation with one or more wavelength in the range from 200 to 1700nm toward at least one tooth.

2. The kit for whitening teeth according to the preceding claim, wherein the ratio of the concentration in weight percent of bleaching agent present in the aqueous phase to the net concentration in weight percent of bleaching agent present in the overall oral composition is at least 100, preferably at least 150, more preferred at least 175, more preferred at least 350.

3. The kit for whitening teeth according to anyone of the preceding claims, wherein the delivery carrier is compatible with oral care substances, transparent or translucent to electromagnetic radiation with one or more wavelengths from about 200 to 1700nm, or a combination thereof, preferably wherein the delivery carrier is a strip or deformable strip, more preferred wherein the delivery carrier comprises a polymer or combination of polymers.

4. The kit for whitening teeth according to anyone of the preceding claims, wherein the delivery carrier is a strip (12) of polyethylene, ethylvinyl acetate, polyesters, ethylvinyl alcohol and combinations thereof, more preferred wherein the carrier is a strip of polyethylene having a film thickness of less than 1mm, more preferred less than 0.1mm, more preferred less than 0.05mm, more preferred wherein the film thickness is in the range of from $1\mu$m to $30\mu$m, more preferred from $5\mu$m to $20\mu$m.

5. The kit according to anyone of the preceding claims, wherein the oral composition is applied to the delivery carrier thereby forming an article (10).

6. The kit for whitening teeth according to anyone of the preceding claims, wherein the oral composition comprises from 1 microgram to 5000 micrograms, preferably from 10 micrograms to 500 micrograms, and more preferred from 50 micrograms to 100 micrograms bleaching agent per square centimeter delivery carrier.

7. The kit for whitening teeth according to anyone of preceding claims 1 to 4 or 6, wherein the delivery carrier is a deformable strip of material having a yield point and thickness such that the deformable strip of material (12) at least partially conforms to the shape of the teeth via permanent deformation under a pressure of less than 250,000 Pascals when the delivery system is pressed there against, preferably wherein the material (12) is polyethylene, ethylvinyl acetate, polyesters, ethylvinyl alcohol wax, polyethylene plastic or combinations thereof, more preferred wherein the deformable strip of material (12) is wax.

8. The kit for whitening teeth according to claims 6 or 7, wherein the article (10) has an overall thickness of less than 1.5mm, preferably wherein the deformable strip of material (12) has a nominal film thickness of less than 1.2 mm, more preferred wherein the deformable strip of material (12) has a nominal film thickness of less than 1.0 mm, more preferred wherein the deformable strip of material (12) has a nominal film thickness of 0.8 mm.

9. The kit for whitening teeth according to anyone of the preceding claims, wherein the aqueous phase is from 0.1% to 0.95% by weight of the oral composition.

10. The kit for whitening teeth according to anyone of the preceding claims, wherein the aqueous phase of the oral composition is distributed in the continuous hydrophobic phase of the oral composition in the form of droplets, preferably wherein the aqueous phase is distributed with a number-average droplet diameter in the range of from 0.01 microns to 1000 microns, more preferred with a number-average droplet diameter in the range from 0.1 micron to 1000 microns, more preferred with a number-average droplet diameter in the range from 1 micron to 100 microns.

11. The kit for whitening teeth according to anyone of the preceding claims, wherein the net amount of the bleaching agent in each region of the aqueous phase is at least 2% by weight of the aqueous phase, preferably wherein the net amount of the bleaching agent in each droplet of the aqueous phase is at least 2% by weight of the aqueous phase.

12. The kit for whitening teeth according to anyone of the preceding claims, wherein the oral composition is substantially free of acids, substantially free of alcohols, substantially free of an emulsifier, substantially free of a stabilizing agent, substantially free of a thickening agent or substantially free of a mixture thereof, preferably wherein the oral composition is free of an emulsifier.

13. The kit for whitening teeth according to anyone of the preceding claims, wherein the oral composition comprises at least 99.1%, preferably at least 99.5% by weight of the continuous, hydrophobic phase.

14. The kit for whitening teeth according to anyone of the preceding claims, wherein the hydrophobic phase of the oral composition is selected from non-toxic edible oils, saturated or unsaturated fatty alcohols, silicones, polysiloxanes, aliphatic hydrocarbons, long chain triglycerides, fatty esters and mixtures thereof, preferably selected from mineral oil, mineral oil thickened with wax, mineral oil thickened with polyethylene, petrolatum and mixtures thereof, preferably wherein the hydrophobic phase is petrolatum.

15. The kit for whitening teeth according to anyone of the preceding claims, wherein the bleaching agent comprises a source of peroxide radicals, preferably is selected from the group consisting of peroxides, metal chlorites, perborates, percarbonates, peroxyacids, persulfates, hydrogen peroxide, urea peroxide, calcium peroxide, carbamide peroxide, and mixtures thereof; more preferred selected from hydrogen peroxide, urea peroxide, calcium peroxide, carbamide peroxide, and mixtures thereof and more preferred wherein the bleaching agent is hydrogen peroxide.

16. The kit for whitening teeth according to anyone of the preceding claim, wherein the net amount of the bleaching agent is smaller than 0.1% by weight of the oral composition, preferably in the range of from 0.01% to 0.095% by weight of the oral composition, more preferred in the range of from 0.05% to 0.09% by weight of the oral composition.

17. The kit for whitening teeth according to anyone of the preceding claims, wherein the bleaching efficacy of the oral care delivery system calculated as $\Delta b*$ is at least 0.25, preferably at least 0.5, more preferred at least 1.0, even more preferred at least 1.5 measured per the clinical protocol as disclosed herein.

18. The kit for whitening teeth according to anyone of the preceding claims, wherein the bleaching efficacy of the oral care delivery system calculated as $-\Delta b*$ is at least 10%, preferably at least 100%, more preferred at least 1000%, even more preferred at least 10000% more than the bleaching efficacy of an oral care composition in form of an aqueous solution or aqueous gel comprising the same bleaching agent at the same overall concentration dissolved into the aqueous solution or aqueous gel measured per the clinical protocol as disclosed herein.

19. The kit for whitening teeth according to anyone of the preceding claims, wherein the ratio of bleaching efficacy of the oral composition calculated as $-\Delta b*$ and measured per the clinical protocol as disclosed herein to the net weight percent of bleaching agent present in the overall oral composition is at least 2.5, preferably at least 5, more preferred at least 10, more preferred at least 15.

20. Kit according to anyone of the preceding claims for whitening teeth and/or removing stain from tooth surfaces.

21. Non-therapeutic cosmetic method for whitening teeth comprising

   a) applying an article according to anyone of claims 5 to 19 to at least one tooth surface such that the delivery carrier contacts the oral care composition against the at least one tooth surface;
   b) letting the oral composition stay on the at least one tooth surface for a period of time, preferably for at least 1 minute, more preferred for at least 10 minutes, even more preferred for at least 30 minutes, and most preferred for at least 60 minutes; and
   c) directing the electromagnetic radiation source emitting electromagnetic radiation with one or more wavelengths in the range from 200nm to 1700nm toward the at least one tooth for a period of time, preferably for at least 1 minute, more preferred for at least 5 minutes, and most preferred for at least 10 minutes.

22. Non-therapeutic cosmetic method for whitening teeth using a kit according to anyone of claims 1 to 4 or 6 to 19 comprising

   a) applying the delivery carrier to at least one tooth such that it substantially conforms to the shape of the at least one tooth;
   b) removing the shaped delivery carrier from the at least one tooth;
   c) applying the oral composition to the shaped delivery carrier;
   d) re-applying the shaped delivery carrier to the at least one tooth such that it conforms to the shape of the at least one tooth and contacts the oral care composition against the at least one tooth surface;
   e) letting the oral composition stay on the at least one tooth surface for a period of time, preferably for at least 1 minute, more preferred for at least 10 minutes, even more preferred for at least 30 minutes, and most preferred for at least 60 minutes; and
   f) directing the electromagnetic radiation source emitting electromagnetic radiation with one or more wavelengths in the range from 200nm to 1700nm toward the at least one tooth for a period of time, preferably for at least 1 minute, more preferred for at least 5 minutes, and most preferred for at least 10 minutes.

23. The kit according to anyone of claims 1 to 19 or the cosmetic method according to claims 20 or 21, wherein the electromagnetic radiation source is capable of emitting radiation with an intensity of the electromagnetic radiation in the range from 200nm to 1700nm, preferably 200nm to 1 100nm, more preferably from 400 nm to 500 nm impinging on the outer surface of the carrier or tooth in the range from 0.1 to 500, preferably in the range from 10 to 250, more preferred in the range from 20 to 150, and most preferred in the range from 175 to 225 mW/cm2 as measured using the procedure described herein.

**Patentansprüche**

1. Kit zum Aufhellen von Zähnen, umfassend:

   I) einen Abgabeträger,
   II) eine orale Zusammensetzung in der Form einer Wasser-in-Öl-Emulsion zum Aufhellen von Zähnen, umfassend

      i) zu 0,01 Gew.-% bis 1 Gew.-% eine wässrige Phase;
      ii) wenigstens 99 Gew.-% eine kontinuierliche, hydrophobe Phase;
      iii) wenigstens einen Mundpflegewirkstoff, der in der wässrigen Phase enthalten ist; wobei der Wirkstoff ein Bleichmittel ist und die Nettokonzentration des Bleichmittels bis zu 0,1 Gew.-% der oralen Zusammensetzung beträgt; und

   wobei die orale Zusammensetzung auf den Abgabeträger aufbringbar ist; und
   III) eine Quelle elektromagnetischer Strahlung, die in der Lage ist, elektromagnetische Strahlung mit einer oder mehreren Wellenlängen im Bereich von 200 nm bis 1700 nm in Richtung wenigstens eines Zahns zu richten.

2. Kit zum Aufhellen von Zähnen nach dem vorstehenden Anspruch, wobei das Verhältnis der Konzentration in Gewichtsprozent des in der wässrigen Phase vorhandenen Bleichmittels zu der Nettokonzentration in Gewichtsprozent des in der gesamten oralen Zusammensetzung vorhandenen Bleichmittels wenigstens 100, vorzugsweise wenigstens 150, mehr bevorzugt wenigstens 175, mehr bevorzugt wenigstens 350 beträgt.

3. Kit zum Aufhellen von Zähnen nach einem der vorstehenden Ansprüche, wobei der Abgabeträger mit Mundpflegestoffen kompatibel ist, gegenüber elektromagnetischer Strahlung mit einer oder mehreren Wellenlängen von etwa 200 bis 1700 nm transparent oder lichtdurchlässig ist, oder einer Kombination davon, wobei der Abgabeträger vorzugsweise ein Streifen oder verformbarer Streifen ist, wobei der Abgabeträger mehr bevorzugt ein Polymer oder eine Kombination von Polymeren umfasst.

4. Kit zum Aufhellen von Zähnen nach einem der vorstehenden Ansprüche, wobei der Abgabeträger ein Streifen (12) aus Polyethylen, Ethylvinylacetat, Polyester, Ethylvinylalkohol und Kombinationen davon ist, wobei der Träger mehr bevorzugt ein Streifen aus Polyethylen mit einer Filmdicke von weniger als 1 mm, mehr bevorzugt weniger als 0,1 mm, mehr bevorzugt weniger als 0,05 mm ist, wobei die Filmdicke mehr bevorzugt im Bereich von 1 $\mu$m bis 30 $\mu$m, mehr bevorzugt von 5 $\mu$m bis 20 $\mu$m, liegt.

5. Kit nach einem der vorstehenden Ansprüche, wobei die orale Zusammensetzung auf den Abgabeträger aufgetragen wird, dadurch einen Artikel (10) bildend.

6. Kit zum Aufhellen von Zähnen nach einem der vorstehenden Ansprüche, wobei die orale Zusammensetzung von 1 Mikrogramm bis 5000 Mikrogramm, vorzugsweise von 10 Mikrogramm bis 500 Mikrogramm, und mehr bevorzugt von 50 Mikrogramm bis 100 Mikrogramm Bleichmittel pro Quadratzentimeter Abgabeträger umfasst.

7. Kit zum Aufhellen von Zähnen nach einem der vorstehenden Ansprüche 1 bis 4 oder 6, wobei der Abgabeträger ein verformbarer Materialstreifen mit einer derartigen Fließgrenze und Dicke ist, dass sich der verformbare Materialstreifen (12) durch dauerhafte Verformung unter einem Druck von weniger als 250.000 Pascal wenigstens teilweise an die Form der Zähne anpasst, wenn das Abgabesystem dort gegen gedrückt wird, wobei das Material (12) vorzugsweise Polyethylen, Ethylvinylacetat, Polyester, Ethylvinylalkoholwachs, Polyethylenkunststoff oder Kombinationen davon ist, wobei der verformbare Materialstreifen (12) mehr bevorzugt Wachs ist.

8. Kit zum Aufhellen von Zähnen nach einem der Ansprüche 6 oder 7, wobei der Artikel (10) eine Gesamtdicke von weniger als 1,5 mm aufweist, wobei der verformbare Materialstreifen (12) vorzugsweise eine nominale Filmdicke von weniger als 1,2 mm aufweist, wobei der verformbare Materialstreifen (12) mehr bevorzugt eine nominale Filmdicke von weniger als 1,0 mm aufweist, wobei der verformbare Materialstreifen (12) mehr bevorzugt eine nominale Filmdicke von 0,8 mm aufweist.

9. Kit zum Aufhellen von Zähnen nach einem der vorstehenden Ansprüche, wobei die wässrige Phase von 0,1 Gew.-% bis 0,95 Gew.-% der oralen Zusammensetzung ausmacht.

10. Kit zum Aufhellen von Zähnen nach einem der vorstehenden Ansprüche, wobei die wässrige Phase der oralen Zusammensetzung in der kontinuierlichen hydrophoben Phase der oralen Zusammensetzung in der Form von Tröpfchen verteilt ist, wobei die wässrige Phase vorzugsweise mit einem zahlengemittelten Tröpfchendurchmesser im Bereich von 0,01 Mikrometer bis 1000 Mikrometer, mehr bevorzugt mit einem zahlengemittelten Tröpfchendurchmesser im Bereich von 0,1 Mikrometer bis 1000 Mikrometer, mehr bevorzugt mit einem zahlengemittelten Tröpfchendurchmesser im Bereich von 1 Mikrometer bis 100 Mikrometer verteilt ist.

11. Kit zum Aufhellen von Zähnen nach einem der vorstehenden Ansprüche, wobei die Nettomenge des Bleichmittels in jeder Region der wässrigen Phase wenigstens 2 Gew.-% der wässrigen Phase beträgt, wobei die Nettomenge des Bleichmittels in jedem Tröpfchen der wässrigen Phase vorzugsweise wenigstens 2 Gew.-% der wässrigen Phase beträgt.

12. Kit zum Aufhellen von Zähnen nach einem der vorstehenden Ansprüche, wobei die orale Zusammensetzung im Wesentlichen frei von Säuren, im Wesentlichen frei von Alkoholen, im Wesentlichen frei von einem Emulgator, im Wesentlichen frei von einem Stabilisierungsmittel, im Wesentlichen frei von einem Verdickungsmittel oder im Wesentlichen frei von einer Mischung davon ist, wobei die orale Zusammensetzung vorzugsweise frei von einem Emulgator ist.

13. Kit zum Aufhellen von Zähnen nach einem der vorstehenden Ansprüche, wobei die orale Zusammensetzung wenigstens 99,1 Gew.-%, vorzugsweise wenigstens 99,5 Gew.-% der kontinuierlichen, hydrophoben Phase, umfasst.

14. Kit zum Aufhellen von Zähnen nach einem der vorstehenden Ansprüche, wobei die hydrophobe Phase der oralen Zusammensetzung ausgewählt ist aus nicht-toxischen Speiseölen, gesättigten oder ungesättigten Fettalkoholen, Silikonen, Polysiloxanen, aliphatischen Kohlenwasserstoffen, langkettigen Triglyceriden, Fettestern und Mischungen davon, vorzugsweise ausgewählt aus Mineralöl, mit Wachs verdicktem Mineralöl, mit Polyethylen, Petrolatum und Mischungen davon verdicktem Mineralöl, wobei die hydrophobe Phase vorzugsweise Petrolatum ist.

15. Kit zum Aufhellen von Zähnen nach einem der vorstehenden Ansprüche, wobei das Bleichmittel eine Quelle von Peroxidradikalen umfasst, die vorzugsweise ausgewählt ist aus der Gruppe bestehend aus Peroxiden, Metallchloriten, Perboraten, Percarbonaten, Peroxysäuren, Persulfaten, Wasserstoffperoxid, Harnstoffperoxid, Calciumperoxid, Carbamidperoxid und Mischungen davon; mehr bevorzugt ausgewählt aus Wasserstoffperoxid, Harnstoffperoxid, Calciumperoxid, Carbamidperoxid und Mischungen davon, und wobei das Bleichmittel mehr bevorzugt Wasserstoffperoxid ist.

16. Kit zum Aufhellen von Zähnen nach einem der vorstehenden Ansprüche, wobei die Nettomenge des Bleichmittels kleiner ist als 0,1 Gew.-% der oralen Zusammensetzung, vorzugsweise im Bereich von 0,01 Gew.-% bis 0,095 Gew.-% der oralen Zusammensetzung, mehr bevorzugt im Bereich von 0,05 Gew.-% bis 0,09 Gew.-% der oralen Zusammensetzung liegt.

17. Kit zum Aufhellen von Zähnen nach einem der vorstehenden Ansprüche, wobei die Bleichwirksamkeit des Mundpflegeabgabesystems, berechnet als $\Delta b^*$, wenigstens 0,25, vorzugsweise wenigstens 0,5, mehr bevorzugt wenigstens 1,0, noch mehr bevorzugt wenigstens 1,5 beträgt, gemessen nach dem hierin offenbarten klinischen Protokoll.

18. Kit zum Aufhellen von Zähnen nach einem der vorstehenden Ansprüche, wobei die Bleichwirksamkeit des Mundpflegeabgabesystems, berechnet als $-\Delta b^*$, wenigstens 10 %, vorzugsweise wenigstens 100 %, mehr bevorzugt wenigstens 1000 %, noch mehr bevorzugt wenigstens 10000 % höher ist als die Bleichwirksamkeit einer Mundpflegezusammensetzung in Form einer wässrigen Lösung oder eines wässrigen Gels, umfassend das gleiche Bleichmittel bei der gleichen Gesamtkonzentration gelöst in der wässrigen Lösung oder dem wässrigen Gel, gemessen nach dem hierin offenbarten klinischen Protokoll.

**19.** Kit zum Aufhellen von Zähnen nach einem der vorstehenden Ansprüche, wobei das Verhältnis der Bleichwirksamkeit der oralen Zusammensetzung, berechnet als -Δb* und gemessen nach dem hierin offenbarten klinischen Protokoll, zu dem Nettogewichtsprozentsatz des in der gesamten oralen Zusammensetzung vorhandenen Bleichmittels wenigstens 2,5, vorzugsweise wenigstens 5, mehr bevorzugt wenigstens 10, mehr bevorzugt wenigstens 15 beträgt.

**20.** Kit nach einem der vorstehenden Ansprüche zum Aufhellen von Zähnen und/oder Entfernen von Verfärbung von Zahnoberflächen.

**21.** Nicht-therapeutisches kosmetisches Verfahren zum Aufhellen von Zähnen, umfassend

a) Aufbringen eines Artikels nach einem der Ansprüche 5 bis 19 auf wenigstens eine Zahnoberfläche, sodass der Abgabeträger mit der Mundpflegezusammensetzung gegen die wenigstens eine Zahnoberfläche in Kontakt kommt;
b) Belassen der oralen Zusammensetzung auf der wenigstens einen Zahnoberfläche für einen Zeitabschnitt, vorzugsweise für wenigstens 1 Minute, mehr bevorzugt für wenigstens 10 Minuten, noch mehr bevorzugt für wenigstens 30 Minuten und am meisten bevorzugt für wenigstens 60 Minuten; und
c) Richten der Quelle elektromagnetischer Strahlung, die elektromagnetische Strahlung mit einer oder mehreren Wellenlängen im Bereich von 200 nm bis 1700 nm emittiert, in Richtung des wenigstens einen Zahns für einen Zeitraum, vorzugsweise für wenigstens 1 Minute, mehr bevorzugt für wenigstens 5 Minuten und am meisten bevorzugt wenigstens 10 Minuten.

**22.** Nicht-therapeutisches, kosmetisches Verfahren zum Aufhellen von Zähnen unter Verwendung eines Kits nach einem der Ansprüche 1 bis 4 oder 6 bis 19, umfassend

a) Aufbringen des Abgabeträgers auf wenigstens einen Zahn, sodass er sich im Wesentlichen der Form des wenigstens einen Zahns anpasst;
b) Entfernen des geformten Abgabeträgers von dem wenigstens einen Zahn;
c) Aufbringen der oralen Zusammensetzung auf den geformten Abgabeträger;
d) erneutes Aufbringen des geformten Abgabeträgers auf den wenigstens einen Zahn, sodass er sich der Form des wenigstens einen Zahns anpasst und mit der Mundpflegezusammensetzung gegen die wenigstens eine Zahnoberfläche in Kontakt kommt;
e) Belassen der oralen Zusammensetzung auf der wenigstens einen Zahnoberfläche für einen Zeitraum, vorzugsweise für wenigstens 1 Minute, mehr bevorzugt für wenigstens 10 Minuten, noch mehr bevorzugt für wenigstens 30 Minuten und am meisten bevorzugt für wenigstens 60 Minuten; und
f) Richten der Quelle elektromagnetischer Strahlung, die elektromagnetische Strahlung mit einer oder mehreren Wellenlängen im Bereich von 200 nm bis 1700 nm emittiert, in Richtung des wenigstens einen Zahns für einen Zeitabschnitt, vorzugsweise für wenigstens 1 Minute, mehr bevorzugt für wenigstens 5 Minuten und am meisten bevorzugt für wenigstens 10 Minuten.

**23.** Kit nach einem der Ansprüche 1 bis 19 oder kosmetisches Verfahren nach Anspruch 20 oder 21, wobei die Quelle elektromagnetischer Strahlung in der Lage ist, Strahlung mit einer Intensität der elektromagnetischen Strahlung im Bereich von 200 nm bis 1700 nm, vorzugsweise 200 nm bis 1100 nm, mehr bevorzugt von 400 nm bis 500 nm zu emittieren, die im Bereich von 0,1 bis 500, vorzugsweise im Bereich von 10 bis 250, mehr bevorzugt im Bereich von 20 bis 150 und am meisten bevorzugt im Bereich von 175 bis 225 mW/cm2, wie gemessen unter Verwendung der hierin beschriebenen Prozedur, auf die Außenoberfläche des Trägers oder Zahns auftrifft.

**Revendications**

**1.** Trousse pour blanchir les dents comprenant :

I) un véhicule d'administration,
II) une composition orale sous la forme d'une émulsion eau-dans-huile pour blanchir les dents, comprenant

i) de 0,01 % à 1 % en poids d'une phase aqueuse ;
ii) au moins 99 % en poids d'une phase hydrophobe continue ;
iii) au moins un agent actif de soins bucco-dentaires compris dans la phase aqueuse ;
dans laquelle l'agent actif est un agent de blanchiment et la concentration nette de l'agent de blanchiment va jusqu'à 0,1 % en poids de la composition orale ; et
dans laquelle la composition orale est applicable sur le véhicule d'administration ; et

III) une source de rayonnement électromagnétique susceptible de diriger un rayonnement électromagnétique avec une ou plusieurs longueurs d'onde dans la plage allant de 200 à 1700 nm vers au moins une dent.

2. Trousse pour blanchir les dents selon la revendication précédente, dans laquelle le rapport de la concentration en pour cent en poids d'agent de blanchiment présent dans la phase aqueuse à la concentration nette en pour cent en poids d'agent de blanchiment présent dans la composition orale globale est d'au moins 100, de préférence au moins 150, plus préférablement au moins 175, plus préférablement au moins 350.

3. Trousse pour blanchir les dents selon l'une quelconque des revendications précédentes, dans laquelle le véhicule d'administration est compatible avec des substances de soins bucco-dentaires, transparent ou translucide à un rayonnement électromagnétique avec une ou plusieurs longueurs d'onde d'environ 200 à 1700 nm, ou une combinaison de ceux-ci, de préférence dans laquelle le véhicule d'administration est une bandelette ou bandelette déformable, plus préférablement dans laquelle le véhicule d'administration comprend un polymère ou une combinaison de polymères.

4. Trousse pour blanchir les dents selon l'une quelconque des revendications précédentes, dans laquelle le véhicule d'administration est une bandelette (12) de polyéthylène, d'acétate d'éthylvinyle, de polyesters, d'alcool éthylvinylique et leurs combinaisons, plus préférablement dans laquelle le véhicule est une bandelette de polyéthylène ayant une épaisseur de film inférieure à 1 mm, plus préférablement inférieure à 0,1 mm, plus préférablement inférieure à 0,05 mm, plus préférablement dans laquelle l'épaisseur de film est dans la plage allant de 1 $\mu$m à 30 $\mu$m, plus préférablement de 5 $\mu$m à 20 $\mu$m.

5. Trousse selon l'une quelconque des revendications précédentes, dans laquelle la composition orale est appliquée au véhicule d'administration en formant de ce fait un article (10).

6. Trousse pour blanchir les dents selon l'une quelconque des revendications précédentes, dans laquelle la composition orale comprend de 1 microgramme à 5000 microgrammes, de préférence de 10 microgrammes à 500 microgrammes, et plus préférablement de 50 microgrammes à 100 microgrammes d'agent de blanchiment par centimètre carré de véhicule d'administration.

7. Trousse pour blanchir les dents selon l'une quelconque des revendications 1 à 4 ou 6 précédentes, dans laquelle le véhicule d'administration est une bandelette déformable de matériau ayant une limite d'élasticité et une épaisseur telles que la bandelette déformable de matériau (12) épouse au moins partiellement la forme des dents par l'intermédiaire d'une déformation permanente sous une pression inférieure à 250 000 Pascals lorsque le système d'administration est pressé contre celles-ci, de préférence dans laquelle le matériau (12) est du polyéthylène, de l'acétate d'éthylvinyle, des polyesters, de la cire d'alcool éthylvinylique, du plastique polyéthylène ou leurs combinaisons, plus préférablement dans laquelle la bandelette déformable de matériau (12) est de la cire.

8. Trousse pour blanchir les dents selon les revendications 6 ou 7, dans laquelle l'article (10) a une épaisseur globale inférieure à 1,5 mm, de préférence dans laquelle la bandelette déformable de matériau (12) a une épaisseur nominale de film inférieure à 1,2 mm, plus préférablement dans laquelle la bandelette déformable de matériau (12) a une épaisseur nominale de film inférieure à 1,0 mm, plus préférablement dans laquelle la bandelette déformable de matériau (12) a une épaisseur nominale de film de 0,8 mm.

9. Trousse pour blanchir les dents selon l'une quelconque des revendications précédentes, dans laquelle la phase aqueuse représente de 0,1 % à 0,95 % en poids de la composition orale.

10. Trousse pour blanchir les dents selon l'une quelconque des revendications précédentes, dans laquelle la phase aqueuse de la composition orale est distribuée dans la phase hydrophobe continue de la composition orale sous la forme de gouttelettes, de préférence dans laquelle la phase aqueuse est distribuée avec un diamètre de gouttelette moyen en nombre dans la plage allant de 0,01 micromètre à 1000 micromètres, plus préférablement avec un diamètre de gouttelette moyen en nombre dans la plage allant de 0,1 micromètre à 1000 micromètres, plus préférablement avec un diamètre de gouttelette moyen en nombre dans la plage allant de 1 micromètre à 100 micromètres.

11. Trousse pour blanchir les dents selon l'une quelconque des revendications précédentes, dans laquelle la quantité nette de l'agent de blanchiment dans chaque région de la phase aqueuse est d'au moins 2 % en poids de la phase aqueuse, de préférence dans laquelle la quantité nette de l'agent de blanchiment dans chaque gouttelette de la phase aqueuse est d'au moins 2 % en poids de la phase aqueuse.

12. Trousse pour blanchir les dents selon l'une quelconque des revendications précédentes, dans laquelle la composition orale est essentiellement dépourvue d'acides, essentiellement dépourvue d'alcools, essentiellement dépourvue d'émulsifiant, essentiellement dépourvue d'un agent stabilisant, essentiellement dépourvue d'un agent épaississant ou essentiellement dépourvue d'un mélange de ceux-ci, de préférence dans laquelle la composition orale est dépourvue d'un émulsifiant.

13. Trousse pour blanchir les dents selon l'une quelconque des revendications précédentes, dans laquelle la composition orale comprend au moins 99,1 %, de préférence au moins 99,5 % en poids de la phase hydrophobe continue.

14. Trousse pour blanchir les dents selon l'une quelconque des revendications précédentes, dans laquelle la phase hydrophobe de la composition orale est choisie parmi des huiles comestibles non toxiques, des alcools gras saturés ou insaturés, des silicones, des polysiloxanes, des hydrocarbures aliphatiques, des triglycérides à longue chaîne, des esters gras et leurs mélanges, choisie de préférence parmi une huile minérale, une huile minérale épaissie avec de la cire, une huile minérale épaissie avec du polyéthylène, de la vaseline et leurs mélanges, de préférence dans laquelle la phase hydrophobe est de la vaseline.

15. Trousse pour blanchir les dents selon l'une quelconque des revendications précédentes, dans laquelle l'agent de blanchiment comprend une source de radicaux peroxyde, de préférence est choisi dans le groupe constitué de peroxydes, chlorites métalliques, perborates, percarbonates, peroxyacides, persulfates, peroxyde d'hydrogène, peroxyde d'urée, peroxyde de calcium, peroxyde de carbamide, et des mélanges de ceux-ci ; plus préférablement choisi parmi le peroxyde d'hydrogène, le peroxyde d'urée, le peroxyde de calcium, le peroxyde de carbamide, et des mélanges de ceux-ci, et plus préférablement dans lequel l'agent de blanchiment est le peroxyde d'hydrogène.

16. Trousse pour blanchir les dents selon l'une quelconque des revendications précédentes, dans laquelle la quantité nette de l'agent de blanchiment est inférieure à 0,1 % en poids de la composition orale, de préférence dans la plage allant de 0,01 % à 0,095 % en poids de la composition orale, plus préférablement dans la plage allant de 0,05 % à 0,09 % en poids de la composition orale.

17. Trousse pour blanchir les dents selon l'une quelconque des revendications précédentes, dans laquelle l'efficacité de blanchiment du système d'administration de soins bucco-dentaires calculée en tant que $\Delta b^*$ est au moins 0,25, de préférence au moins 0,5, plus préférablement au moins 1,0, encore plus préférablement au moins 1,5 mesurée par le protocole clinique tel que décrit ici.

18. Trousse pour blanchir les dents selon l'une quelconque des revendications précédentes, dans laquelle l'efficacité de blanchiment du système d'administration de soins bucco-dentaires calculée en tant que $-\Delta b^*$ est au moins 10 %, de préférence au moins 100 %, plus préférablement au moins 1000 %, même plus préférablement au moins 10 000 % supérieure à l'efficacité de blanchiment d'une composition de soins bucco-dentaires sous forme de solution aqueuse ou de gel aqueux comprenant le même agent de blanchiment à la même concentration globale, dissous dans la solution aqueuse ou le gel aqueux, mesurée par le protocole clinique tel que décrit ici.

19. Trousse pour blanchir les dents selon l'une quelconque des revendications précédentes, dans laquelle le rapport de l'efficacité de blanchiment de la composition orale calculé en tant que $-\Delta b^*$ et mesurée par le protocole clinique tel que décrit ici au pourcentage en poids net d'agent de blanchiment présent dans la composition orale globale est d'au moins 2,5, de préférence au moins 5, plus préférablement au moins 10, plus préférablement au moins 15.

20. Trousse selon l'une quelconque des revendications précédentes pour blanchir les dents et/ou éliminer les taches de surfaces dentaires.

21. Procédé cosmétique non thérapeutique pour blanchir les dents comprenant

a) l'application d'un article selon l'une quelconque des revendications 5 à 19 à au moins une surface dentaire de telle sorte que le véhicule d'administration vient en contact avec la composition de soins bucco-dentaires contre l'au moins une surface dentaire ;
b) le fait de laisser la composition orale rester sur l'au moins une surface dentaire pendant un laps de temps, de préférence pendant au moins 1 minute, plus préférablement pendant au moins 10 minutes, encore plus préférablement pendant au moins 30 minutes, et le plus préférablement pendant au moins 60 minutes ; et

c) l'orientation de la source de rayonnement électromagnétique émettant un rayonnement électromagnétique avec une ou plusieurs longueurs d'onde dans la plage allant de 200 nm à 1700 nm en direction de l'au moins une dent pendant un laps de temps, de préférence pendant au moins 1 minute, plus préférablement pendant au moins 5 minutes et le plus préférablement pendant au moins 10 minutes.

22. Procédé cosmétique non thérapeutique pour blanchir les dents en utilisant une trousse selon l'une quelconque des revendications 1 à 4 ou 6 à 19 comprenant

a) l'application du véhicule d'administration à au moins une dent de telle sorte qu'il épouse essentiellement la forme de l'au moins une dent ;

b) l'enlèvement du véhicule d'administration mis en forme de l'au moins une dent ;

c) l'application de la composition orale au véhicule d'administration mis en forme ;

d) la réapplication du véhicule d'administration mis en forme à l'au moins une dent de telle sorte qu'il épouse la forme de l'au moins une dent et met en contact la composition de soins bucco-dentaires contre l'au moins une surface dentaire ;

e) le fait de laisser la composition orale rester sur l'au moins une surface dentaire pendant un laps de temps, de préférence pendant au moins 1 minute, plus préférablement pendant au moins 10 minutes, encore plus préférablement pendant au moins 30 minutes, et le plus préférablement pendant au moins 60 minutes ; et

f) l'orientation de la source de rayonnement électromagnétique émettant un rayonnement électromagnétique avec une ou plusieurs longueurs d'onde dans la plage allant de 200 nm à 1700 nm en direction de l'au moins une dent pendant un laps de temps, de préférence pendant au moins 1 minute, plus préférablement pendant au moins 5 minutes et le plus préférablement pendant au moins 10 minutes.

23. Trousse selon l'une quelconque des revendications 1 à 19 ou procédé cosmétique selon les revendications 20 ou 21, dans lequel la source de rayonnement électromagnétique est susceptible d'émettre un rayonnement avec une intensité du rayonnement électromagnétique dans la plage allant de 200 nm à 1700 nm, de préférence 200 nm à 1100 nm, plus préférablement de 400 nm à 500 nm arrivant sur la surface externe du véhicule ou de la dent dans la plage de 0,1 à 500, de préférence dans la plage allant de 10 à 250, plus préférablement dans la plage allant de 20 à 150, et le plus préférablement dans la plage allant de 175 à 225 mW/cm2 telle que mesurée en utilisant la procédure décrite ici.

3 3

14

12

10

Fig. 1

10

14

Fig. 2

12

6

10

6

22

Fig. 3

20

10

22

Fig. 4

**Fig. 5**

**Fig. 6**

**Fig. 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6136297 A
- US 6096328 A
- US 5894017 A
- US 5891453 A
- US 5879691 A, Sagel
- US 5989569 A
- US 6045811 A, Dirksing
- WO 2005058267 A1
- WO 2005058268 A1
- US 20070280894 A1, Romano
- US 3988433 A
- US 4051234 A
- US 3959458 A
- US 2798053 A
- US 3506720 A
- EP 0251591 A
- US 20140178443 A1
- WO 03015656 A
- US 2835628 A
- US 20130295525 A

**Non-patent literature cited in the description**

- Encyclopedia of Polymer Science and Engineering. John Wiley & Sons Inc, 1989, vol. 15, 204-308
- **WALTER NOLL.** Chemistry and Technology of Silicones. Academic Press Inc, (Harcourt Brue Javanovich, Publishers, 1968, 282-287, 409-426
- Chemistry and Technology of Lubricants. Chapman and Hall, 1997
- Kirk & Othmer, Encyclopedia of Chemical Technology. Wiley-Interscience Publishers, 1996, vol. 18, 685-707
- The Merck Index. 1989, 1529
- Drug Facts and Comparisons (loose leaf drug information service). Wolters Kluer Company, 1997, 10-17
- Kirk-Othmer, Encyclopedia of Chemical Technology. vol. 21, 207-218
- Remmingtion: the Science and Practice of Pharmacy. vol. II
- Remington: The Science and Practice of Pharmacy. 1995, vol. 1, 282-283